# EUROPEAN PATENT APPLICATION

(11) **EP 2 592 049 A2**
(43) Date of publication of application: **15.05.2013**
(21) Application number: 11803735.7
(22) Date of filing: 07.06.2011
(51) Int. Cl.: C01B 39/48, B01J 29/06, B01J 29/89, C07C 1/20

(54) **ZEOLITE OR AN ANALOGOUS MATERIAL THEREOF INCLUDING MESOPORES ARRANGED REGULARLY OR IRREGULARLY, AND PREPARATION METHOD FOR SAME**

(30) Priority: 05.07.2010 KR 20100064200
(71) Applicant: Kaist, Daejeon 305-701 (KR)
(72) Inventor: RYOO, Ryong, Daejeon 305-345 (KR); JO, Chang Bum, Daejeon 305-701 (KR); NA, Kyung Su, Daejeon 305-701 (KR); KIM, Jeong Nam, Seogwipo-si Jeju-do 697-855 (KR); JUNG, Jin Hwan, Daejeon 305-701 (KR)
(74) Representative: Buchet, Anne
(86) International application number: PCT/KR2011/004128
(87) International publication number: WO 2012/005449

(57) **Abstract**

The present invention relates to a novel zeolite or zeolite-like material synthesized using a zeolite synthesis composition comprising a specifically designed organic surfactant, wherein the zeolite or zeolite-like material comprises a crystalline framework having a thickness corresponding to up to 10 single unit cells along at least one axis, and 2-50 nm mesopores formed by organic assembly of the crystalline framework are regularly or irregularly arranged in the zeolite or zeolite-like material. In addition, the present invention presents a micro-mesoporous molecular sieve material activated or functionalized by dealumination, ion exchange or other post-treatment processes, and a method of using the molecular sieve material as a catalyst. The disclosed novel materials have a significantly increased outer surface area and pore volume due to a combination of micropores and mesopores, and thus show an increased diffusion of molecules therein. Accordingly, these materials will exhibit significantly increased activities compared to conventional zeolite catalysts and ion exchange resins.

## Description

### Technical Field

The present invention relates to a novel zeolite or zeolite-like material synthesized using a zeolite synthesis composition comprising a specifically designed organic surfactant, wherein the zeolite or zeolite-like material comprises a crystalline framework having a thickness corresponding to up to 10 single unit cells along at least one axis, and 2-50 nm mesopores formed by organic assembly of the crystalline framework are regularly or irregularly arranged in the zeolite or zeolite-like material. More specifically, the present invention relates to a novel zeolite material which comprises micropores having a size of 2 nm or less and in which macropores formed by organic assembly of a framework having a uniform thickness corresponding to up to 10 single unit cells are regularly or irregularly arranged, a preparation method thereof, and the catalytic application of the above zeolite or zeolite-like molecular sieve. The present invention also includes a micro-mesoporous molecular sieve material activated or functionalized by impregnation with metal particles, ion exchange, or other post-treatment processes, and the catalytic use thereof.

### Background Art

Zeolites are crystalline microporous materials (pore size: < 2 nm) composed of an aluminosilicate framework. In zeolites, tetrahedral silicon and aluminum atoms are connected to each other by oxygen atoms, and micropores having various sizes and shapes depending to the type of connection are formed. In addition, the ion-exchange capacity and acidity of zeolites can be controlled by controlling the contents of aluminum and silicon atoms in the framework (C. S. Cundy et al., Chem. Rev. 2003, 103, 663). Due to such characteristics, zeolites are frequently used as molecular selective adsorbents, heterogeneous catalysts, and supports. However, due to the small pore size of zeolite, the migration of reactants or products in the pores of zeolite is greatly limited so that the activity and life of the catalyst greatly decrease. To overcome this problem, there was an attempt to facilitate the accessibility of reactants to active sites by increasing the pore size of zeolites.

The size of micropores of zeolite is defined by the number of aluminum and silicon atoms in the micropores. Generally, the micropores of zeolites include up to 12 aluminum and silicon atoms, and enlarging the micropores means increasing the number of atoms in the micropores. Previously, the enlargement of micropores in transition metal-substituted aluminosilicates different from aluminophosphates was reported (Zeolites, 1990, 10, 522-524, Nature, 1988, 331, 698-699). Since then, US Patent Nos. 5,098,684 and 5,102,643 reported that a synthetic, ordered mesoporous silica material including mesopores having a size of 2-50 nm has a significantly increased pore size. However, the previously reported materials could not be used in hydrocarbon cracking reactions and the like which require strong acids, because the acidity, thermal stability and hydrothermal stability thereof are lower than those of conventional zeolites. Particularly, because the ordered mesoporous silica material is comprised of an amorphous silica framework, it is known to have low acidity and very low thermal stability and hydrothermal stability compared to zeolites. Accordingly, there is an urgent need for the development of a novel catalyst which includes mesopores having a uniform size of 2-50 nm while maintaining the acidity or stability of conventional zeolites.

### Disclosure

### Technical Problem

To synthesize a catalyst, an adsorbent and a separating agent, the synthesis of a zeolite having a large pore size is essential. Accordingly, the present inventors have conducted studies to increase the micropore size of zeolites to 2 nm or more.

Zeolites or zeolite-like materials which have a sheet structure have a shortcoming in that when an organic surfactant that supports the sheets is removed by calcination in order to use the zeolites as catalysts, the regularity of the sheet structure is destroyed.

Accordingly, the present inventors have attempted to overcome this shortcoming and develop a novel zeolite or zeolite-like material which has a hexagonally or cubically ordered mesoporous structure or a disordered mesoporous structure. Specifically, the present inventors have designed an organic surfactant that contains ammonium functional groups, which direct a zeolite framework, in the hydrocarbon chain, and have used the organic surfactant to direct the growth of crystals having a thickness corresponding to a single unit cell into structures other than a lamellar structure. In other words, it is an object of the present invention to prepare a zeolite wherein crystals comprising regularly arranged micropores and having a thickness corresponding to a single unit cell are assembled in various manners to form macropores which are regularly or irregularly arranged.

According to the present invention, in addition to zeolites, zeolite-like materials, including aluminophosphate and titanosilicate, can be synthesized by suitably changing the structure of the organic surfactant. In addition, the catalytic application of catalyst materials synthesized according to the present invention is included in the objects of the present invention.

### Technical Solution

To achieve the above objects, the present invention provides a zeolite or zeolite-like material comprising: a crystalline framework which comprises micropores having a size of 2 nm or less and has a thickness corresponding to up to 10 single unit cells; and mesopores formed by self-assembly of the crystalline framework and having a size of 2 nm or more.

Herein, the thickness of the crystalline framework refers to the thickness along at least one axis. Also, "having a thickness corresponding to up to 10 single unit cells" refers to "having a thickness corresponding to greater than 0 but not greater than 10 single unit cells." The reason why "greater than 0" is described is because the present invention also includes the lowest limit of the attainable thickness range. In the present invention, a zeolite material comprised of a crystalline framework having a much smaller thickness than that of a single unit cell was also prepared.

As used herein, the term "zeolite-like material" refers to a material having a structure similar to that of zeolite. The zeolite-like material may comprise a metal element selected from the group consisting of Be, B, Al, Ti, Fe, Ga, V, Cr, Co, Ni, Cu, Zn, Ge, Zr, Nb, Sb, La, Hf and Bi, and typical examples thereof include pure silicate, titanosilicate and aluminophosphate.

The mesopores may be hexagonally ordered mesopores, cubically ordered mesopores, or disordered mesopores.

The zeolite and the zeolite-like material preferably have a BET specific surface area of 600-1500 m²/g, a micropore volume of 0.01-0.20 mL/g, and a mesopore volume of 0.1-3.0 mL/g.

The present invention also provides a material formed by activating or modifying the above zeolite or zeolite-like material using post-treatment such as dealumination, basic aqueous solution treatment, ion exchange, metal incorporation or organic functionalization.

The present invention also provides a method for preparing a crystalline molecular sieve, comprising the steps of: A) polymerizing an organic surfactant of the following formula 1 with an inorganic precursor to form an organic-inorganic hybrid gel comprising nanometer-sized inorganic gel domains stabilized by the organic surfactant; B) converting the nanometer-sized inorganic gel domains to a zeolite or zeolite-like material by a crystallizing process; and C) selectively removing the organic surfactant from the material obtained in step B): 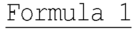 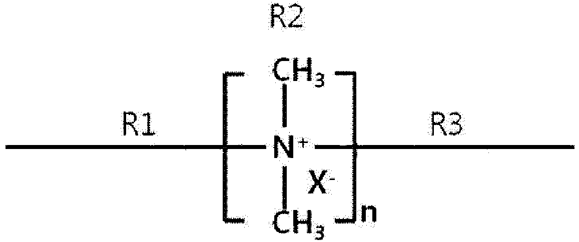 wherein is X⁻ is a halogen anion (Cl⁻, Br⁻, I⁻, etc.) or a hydroxide anion (OH⁻); R1 and R3 are each independently a substituted or unsubstituted alkyl group; R2 is a repeating moiety containing ammonium functional groups; n is the number of ammonium functional groups and is 3 or more; the ammonium functional groups are connected to each other by an alkyl group formed of a hydrocarbon having 3 to 8 carbon atoms; and two methyl (-CH₃) functional groups connected to the ammonium functional group may be substituted with alkyl hydrocarbons having different carbon numbers, such as ethyl (-CH₂CH₃) and propyl (-CH₂CH₂CH₃), or various organic functional groups.

The inorganic precursor may typically be silica or alumina.

Step A) may comprise adding another surfactant, a polymer, an inorganic salt or an organic additive to control the size of mesopores in the range of 2-50 nm.

The crystallizing process may be performed using, for example, hydrothermal synthesis, microwave heating or dry-gel synthesis.

The method for preparing the crystalline molecular sieve may further comprise a step of activating or modifying the material, obtained in step C), using post-treatment such as dealumination, basic aqueous solution treatment, ion exchange, metal incorporation or organic functionalization.

The present invention also provides a crystalline molecular sieve prepared by the above method.

The present invention also provides a process comprising catalytically reforming a hydrocarbon or a substituted form thereof using the above zeolite or zeolite-like material as a catalyst.

In the process, the hydrocarbon may be in a gas phase, a liquid phase, a solid phase, or a mixture thereof.

### Advantageous Effects

As described and demonstrated above, the present invention relates to a zeolite or a zeolite-like material prepared by functionalizing an organic surfactant molecule for forming micropores of a specific zeolite, and using the functionalized molecule to direct a crystalline zeolite framework including micropores having a size of 2 nm or less, and directing 2-50 nm mesopores by self-assembly of the framework in an aqueous solution of the organic surfactant, and to a preparation method thereof.

More specifically, the zeolite material prepared in the present invention is a novel material comprising: a crystalline framework which comprises micropores having a size of 2 nm or less and has a very small thickness corresponding to 1-10 single unit cells; and hexagonally or cubically ordered mesopores or disordered mesopores formed by self-assembly of the framework.

Particularly, the present invention scientifically presents a method for preparing this novel material. The use of this preparation method can prepare aluminosilicate zeolites having an MFI, BEA or MTW microporous structure, as well as zeolites having various frameworks and various microporous structures.

The zeolite and zeolite-like materials prepared according to the present invention possess strong active sites not only in micropores but also in mesopores, and these materials are comprised of a framework having a very small thickness, and thus have a significantly increased specific surface area and pore volume. Thus, these materials will show significantly excellent adsorbent properties and catalytic activities and long life compared to conventional catalysts. In addition, these materials will be highly useful in various fields that use zeolites as catalysts, including the adsorption of organic macromolecules which cannot be adsorbed into micropores, and petroleum reforming reactions.

### Description of Drawings

FIG. 1 shows scanning electron microscope (SEM) images after calcination of a hexagonally ordered mesoporous MFI aluminosilicate prepared according to Example 1 of the present invention.
FIG. 2 shows transmission electron microscope (TEM) images after calcination of a hexagonally ordered mesoporous MFI aluminosilicate prepared according to Example 1 of the present invention.
FIG. 3 shows low-angle powder X-ray diffraction (XRD) data after calcination of a hexagonally ordered mesoporous MFI aluminosilicate prepared according to Example 1 of the present invention.
FIG. 4 shows high-angle powder X-ray diffraction (XRD) data after calcination of a hexagonally ordered mesoporous MFI aluminosilicate prepared according to Example 1 of the present invention.
FIG. 5 shows the ²⁹Si MAS NMR spectrum after calcination of a hexagonally ordered mesoporous MFI aluminosilicate prepared according to Example 1 of the present invention.
FIG. 6 shows the ²⁷Al MAS NMR spectrum after calcination of a hexagonally ordered mesoporous MFI aluminosilicate prepared according to Example 1 of the present invention.
FIG. 7 shows the argon adsorption isotherm and pore size distribution curve after calcination of a hexagonally ordered mesoporous MFI aluminosilicate prepared according to Example 1 of the present invention.
FIG. 8 shows the nitrogen adsorption isotherm and pore size distribution curve after calcination of a hexagonally ordered mesoporous MFI aluminosilicate prepared according to Example 1 of the present invention.
FIG. 9 shows the nitrogen adsorption isotherm and pore size distribution curve of a mesoporous carbon material which is a replica of a hexagonally ordered mesoporous MFI aluminosilicate prepared according to Example 1 of the present invention.
FIG. 10 shows scanning electron microscope (SEM) images after calcination of a hexagonally ordered mesoporous MFI aluminosilicate prepared according to Example 2 of the present invention.
FIG. 11 shows transmission electron microscope (TEM) images after calcination of a hexagonally ordered mesoporous MFI aluminosilicate prepared according to Example 2 of the present invention.
FIG. 12 shows low-angle powder X-ray diffraction (XRD) data after calcination of a hexagonally ordered mesoporous MFI aluminosilicate prepared according to Example 2 of the present invention.
FIG. 13 shows high-angle powder X-ray diffraction (XRD) data after calcination of a hexagonally ordered mesoporous MFI aluminosilicate prepared according to Example 2 of the present invention.
FIG. 14 shows the nitrogen adsorption isotherm and pore size distribution curve after calcination of a hexagonally ordered mesoporous MFI aluminosilicate prepared according to Example 2 of the present invention.
FIG. 15 shows scanning electron microscope (SEM) images after calcination of a hexagonally ordered mesoporous MFI aluminosilicate prepared according to Example 3 of the present invention.
FIG. 16 shows transmission electron microscope (TEM) images after calcination of a hexagonally ordered mesoporous MFI aluminosilicate prepared according to Example 3 of the present invention.
FIG. 17 shows low-angle powder X-ray diffraction (XRD) data after calcination of a hexagonally ordered mesoporous MFI aluminosilicate prepared according to Example 3 of the present invention.
FIG. 18 shows high-angle powder X-ray diffraction (XRD) data after calcination of a hexagonally ordered mesoporous MFI aluminosilicate prepared according to Example 3 of the present invention.
FIG. 19 shows the nitrogen adsorption isotherm and pore size distribution curve after calcination of a hexagonally ordered mesoporous MFI aluminosilicate prepared according to Example 3 of the present invention.
FIG. 20 shows the UV spectrum after calcination of a hexagonally ordered mesoporous MFI titanosilicate prepared according to Example 5 of the present invention.
FIG. 21 shows scanning electron microscope (SEM) images after calcination of a hexagonally ordered mesoporous BEA aluminosilicate prepared according to Example 6 of the present invention.
FIG. 22 shows transmission electron microscope (TEM) images after calcination of a hexagonally ordered mesoporous BEA aluminosilicate prepared according to Example 6 of the present invention.
FIG. 23 shows low-angle powder X-ray diffraction (XRD) data after calcination of a hexagonally ordered mesoporous BEA aluminosilicate prepared according to Example 6 of the present invention.
FIG. 24 shows high-angle powder X-ray diffraction (XRD) data after calcination of a hexagonally ordered mesoporous BEA aluminosilicate prepared according to Example 6 of the present invention.
FIG. 25 shows the ²⁹Si MAS NMR spectrum after calcination of a hexagonally ordered mesoporous BEA aluminosilicate prepared according to Example 6 of the present invention.
FIG. 26 shows the argon adsorption isotherm and pore size distribution curve after calcination of a hexagonally ordered mesoporous BEA aluminosilicate prepared according to Example 6 of the present invention.
FIG. 27 shows the nitrogen adsorption isotherm and pore size distribution curve after calcination of a hexagonally ordered mesoporous BEA aluminosilicate prepared according to Example 6 of the present invention.
FIG. 28 shows scanning electron microscope (SEM) images after calcination of a disordered mesoporous BEA aluminosilicate prepared according to Example 8 of the present invention.
FIG. 29 shows transmission electron microscope (TEM) images after calcination of a disordered mesoporous BEA aluminosilicate prepared according to Example 8 of the present invention.
FIG. 30 shows low-angle powder X-ray diffraction (XRD) data after calcination of a disordered mesoporous BEA aluminosilicate prepared according to Example 8 of the present invention.
FIG. 31 shows high-angle powder X-ray diffraction (XRD) data after calcination of a disordered mesoporous BEA aluminosilicate prepared according to Example 8 of the present invention.
FIG. 32 shows the ²⁹Si MAS NMR spectrum after calcination of a disordered mesoporous BEA aluminosilicate prepared according to Example 8 of the present invention.
FIG. 33 shows the argon adsorption isotherm and pore size distribution curve after calcination of a disordered mesoporous BEA aluminosilicate prepared according to Example 8 of the present invention.
FIG. 34 shows the nitrogen adsorption isotherm and pore size distribution curve after calcination of a disordered mesoporous BEA aluminosilicate prepared according to Example 8 of the present invention.
FIG. 35 shows the nitrogen adsorption isotherm and pore size distribution curve of a mesoporous carbon material which is a replica of a disordered mesoporous BEA aluminosilicate prepared according to Example 8 of the present invention.
FIG. 36 shows scanning electron microscope (SEM) images after calcination of a disordered mesoporous BEA aluminosilicate prepared according to Example 9 of the present invention.
FIG. 37 shows transmission electron microscope (TEM) images after calcination of a disordered mesoporous BEA aluminosilicate prepared according to Example 9 of the present invention.
FIG. 38 shows low-angle powder X-ray diffraction (XRD) data after calcination of a disordered mesoporous BEA aluminosilicate prepared according to Example 9 of the present invention.
FIG. 39 shows high-angle powder X-ray diffraction (XRD) data (after calcination of a disordered mesoporous BEA aluminosilicate prepared according to Example 9 of the present invention.
FIG. 40 shows the nitrogen adsorption isotherm and pore size distribution curve after calcination of a disordered mesoporous BEA aluminosilicate prepared according to Example 9 of the present invention.
FIG. 41 shows scanning electron microscope (SEM) images after calcination of a disordered mesoporous BEA aluminosilicate prepared according to Example 10 of the present invention.
FIG. 42 shows transmission electron microscope (TEM) images after calcination of a disordered mesoporous BEA aluminosilicate prepared according to Example 10 of the present invention.
FIG. 43 shows low-angle powder X-ray diffraction (XRD) data after calcination of a disordered mesoporous BEA aluminosilicate prepared according to Example 10 of the present invention.
FIG. 44 shows high-angle powder X-ray diffraction (XRD) data after calcination of a disordered mesoporous BEA aluminosilicate prepared according to Example 10 of the present invention.
FIG. 45 shows the nitrogen adsorption isotherm and pore size distribution curve after calcination of a disordered mesoporous BEA aluminosilicate prepared according to Example 10 of the present invention.
FIG. 46 shows the UV spectrum after calcination of a disordered mesoporous BEA aluminosilicate prepared according to Example 12 of the present invention.
FIG. 47 shows the nitrogen adsorption isotherm and pore size distribution curve after calcination of a disordered mesoporous BEA aluminosilicate prepared according to Example 13 of the present invention.
FIG. 48 shows low-angle powder X-ray diffraction (XRD) data after calcination of a disordered mesoporous MTW aluminosilicate prepared according to Example 14 of the present invention.
FIG. 49 shows high-angle powder X-ray diffraction (XRD) data after calcination of a disordered mesoporous MTW aluminosilicate prepared according to Example 14 of the present invention.
FIG. 50 shows the nitrogen adsorption isotherm and pore size distribution curve after calcination of a disordered mesoporous MTW aluminosilicate prepared according to Example 14 of the present invention.

### Mode for Invention

Hereinafter, the present invention will be described in detail. In the following description, a detailed description of known functions or constructions will be described.

The terms or words used in the specification and claims should not be limitedly interpreted as normal or lexical meanings, but should be interpreted as meanings and concepts coinciding to technical concepts of the present invention.

Embodiments described in the specification and constructions illustrated in the drawings are only the most preferred example of the present invention, and do not represent all of the technical concepts of the present invention, and thus it should be understood that various equivalents and modifications may be present which can replace them at the time of application of the present invention.

In the present invention, an organic surfactant containing several ammonium functional groups and various organic functional groups was designed and added to a zeolite synthesis gel which was then crystallized under basic or neutral conditions. Then, the organic material was selectively removed by high-temperature calcination or chemical treatment, thereby synthesizing a zeolite or zeolite-like material which comprises micropores together with mesopores formed by assembly of crystals having a thickness corresponding to up to 10 single unit cells.

Herein, the mesopores may be arranged regularly or irregularly depending on an environment and composition for synthesis.

Herein, the zeolite-like material is intended to include the novel zeolite material developed in the present invention, and materials comprising a zeolite framework comprised of aluminophosphate or titanosilicate.

Further, a material obtained by subjecting the zeolite or zeolite-like material to conventional post-treatment, such as dealumination, alkaline treatment, or cation exchange, also falls within the scope of the present invention.

Hereinafter, each step of a method for preparing a novel zeolite and zeolite-like material according to the present invention will be described in further detail.

Step 1: The organic surfactant synthesized as described above is polymerized with an inorganic precursor to form an organic-inorganic hybrid gel. In this case, hydrophobic organic domains are formed between inorganic domains by noncovalent force such as van der Waals force, dipole-dipole interaction, ionic interaction, etc. Herein, the gel domains arranged regularly or irregularly depending on the structure or concentration of the organic material.

Step 2: Then, the nanosized organic-inorganic hybrid gel stabilized by the organic domains are converted to zeolites having various structures by a crystallizing process. Herein, a zeolite framework comprising micropores and having a thickness corresponding to up to 10 single unit cells depending on the structure of the organic surfactant and the composition of gel is formed, and the framework is self-assembled to form macropores. The macropores are also arranged regularly or irregularly depending on the structure of the organic surfactant and the composition of the gel. The crystallizing process may be performed by any conventional methods, including hydrothermal synthesis, dry-gel synthesis, microwave synthesis and the like.

Step 3: The crystallized zeolite may be collected by a conventional method such as filtration or centrifugation. The material thus obtained may be subjected to calcination or other chemical reactions to selectively remove the organic material in total or in part.

The organic surfactant used in the present invention may be represented by the following formula 1: 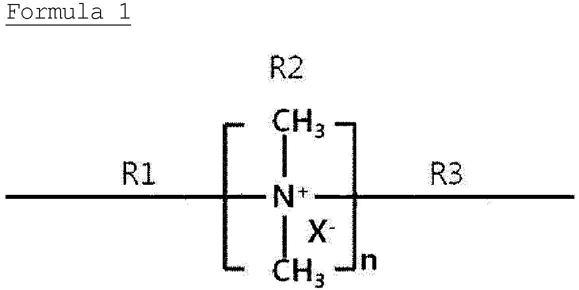 wherein is X⁻ is a halogen anion (Cl⁻, Br⁻, I⁻, etc.) or a hydroxide anion (OH⁻); R1 and R3 are each independently a substituted or unsubstituted alkyl group; R2 is a repeating moiety containing ammonium functional groups; n is the number of ammonium functional groups and is 3 or more; and the ammonium functional groups are connected to each other by an alkyl group which may consist of a hydrocarbon having 3 to 8 carbon atoms and may be substituted with various organic functional groups.

Herein, the structure of the resulting micropores and mesopores can vary depending on the number of the ammonium functional groups, the hydrocarbon chain length of R1 and R3, and the kind of organic functional group. In particular, it was first found in the present invention that the structure and regularity of arrangement of micropores and mesopores can be changed by changing the structure of the organic surfactant.

In the present invention, the organic surfactant is expressed in a general form of R1-nN-R3 depending on the lengths of R1 and R3 and the number (n) of repeating ammonium functional groups (C2). For example, 22-3N-18 means that R1 is a hydrocarbon chain having 22 carbon atoms, R3 is a hydrocarbon chain having 18 carbon atoms and the number of repeating ammonium functional groups is 3. When X is not a halogen, but hydroxide, the expression (OH⁻) follows the general expression.

Meanwhile, two methyl (-CH₃) functional groups connected to the ammonium functional group may be substituted with either alkyl hydrocarbons having different lengths, such as ethyl (-CH₂CH₃) or propyl (-CH₂CH₂CH₃), or various organic functional groups.

Hereinafter, preferred examples will be presented for a better understanding of the present invention. It is to be understood, however, that these examples are for illustrative purposes only and those skilled in the art will appreciate that various modifications, additions and substitutions are possible, without departing from the scope and spirit of the invention as disclosed in the accompanying claims.

### Example 1: Synthesis of aluminosilicate wherein a crystalline microporous MFI zeolite framework is assembled to form mesopores which are hexagonally arranged

Organic surfactant 22-3N-18 (organic surfactant of formula 1 having 22 carbon atoms in C1, 18 carbon atoms in C2, and three ammonium functional groups; CH₃(CH₂)₂₁-N⁺(CH₃)₂-(CH₂)⁶-N⁺(CH₃)₂ -(CH₂)₆-(CH₂)₁₇CH₃·3Br⁻) was mixed with tetraethylorthosilicate (TEOS), NaOH, NaAlO₂ and distilled water to prepare a gel mixture having the following molar composition:
0.5 Al₂O₃: 6.67 Na₂O: 30 SiO₂: 1066 H₂O: 1.3 22-3N-18 organic surfactant.

After strongly stirring the gel mixture with a stirrer at 60 °C for 3 hours, the resulting mixture was placed and maintained in a stainless autoclave at 140 □ for 3 days. After cooling the autoclave to room temperature, the product was filtered and washed several times with distilled water. The resulting product was dried at 110 □, and then calcined at 550 °C for 4 hours to remove the organic surfactant.

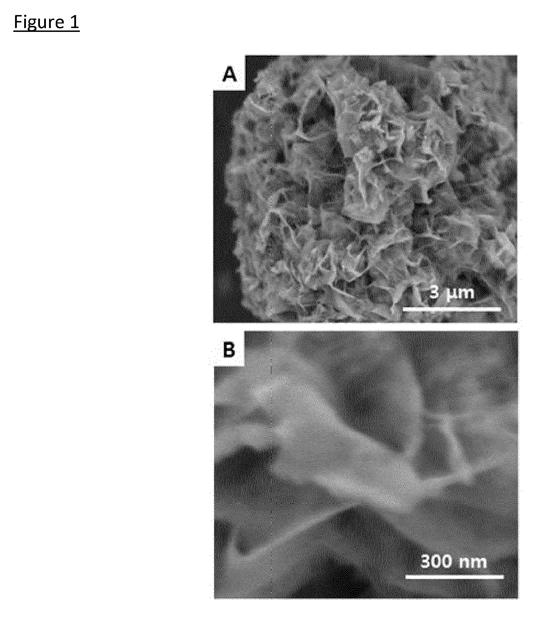

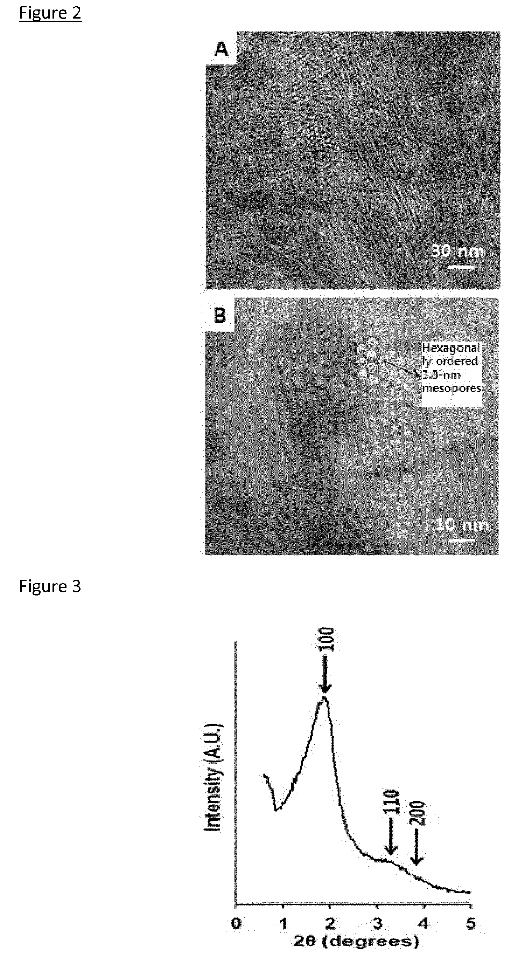

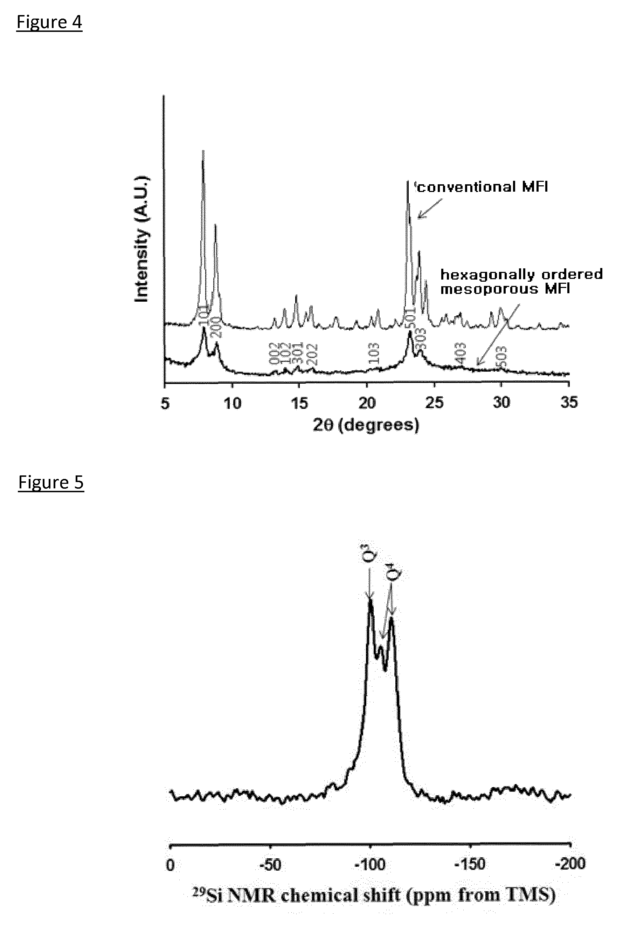

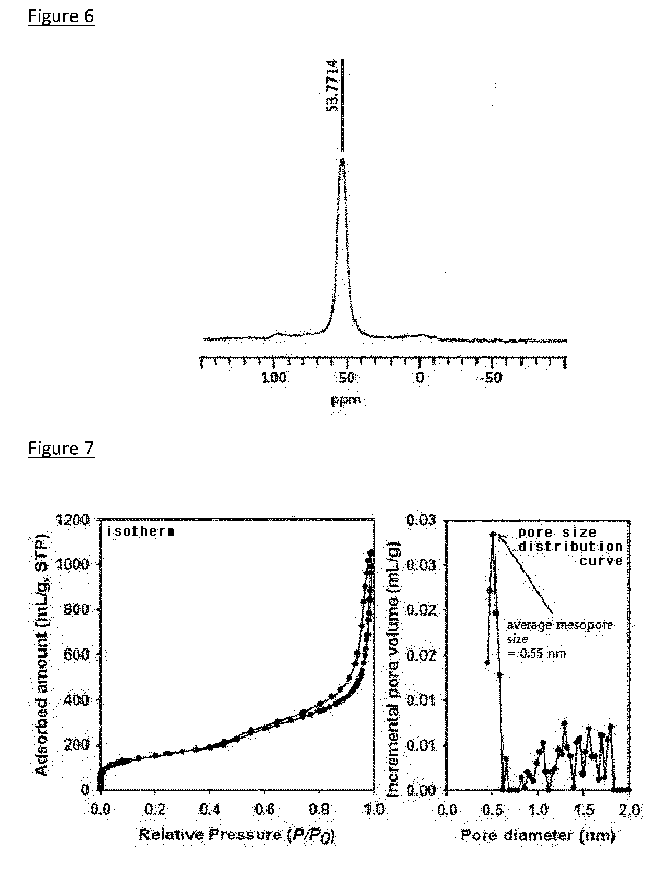

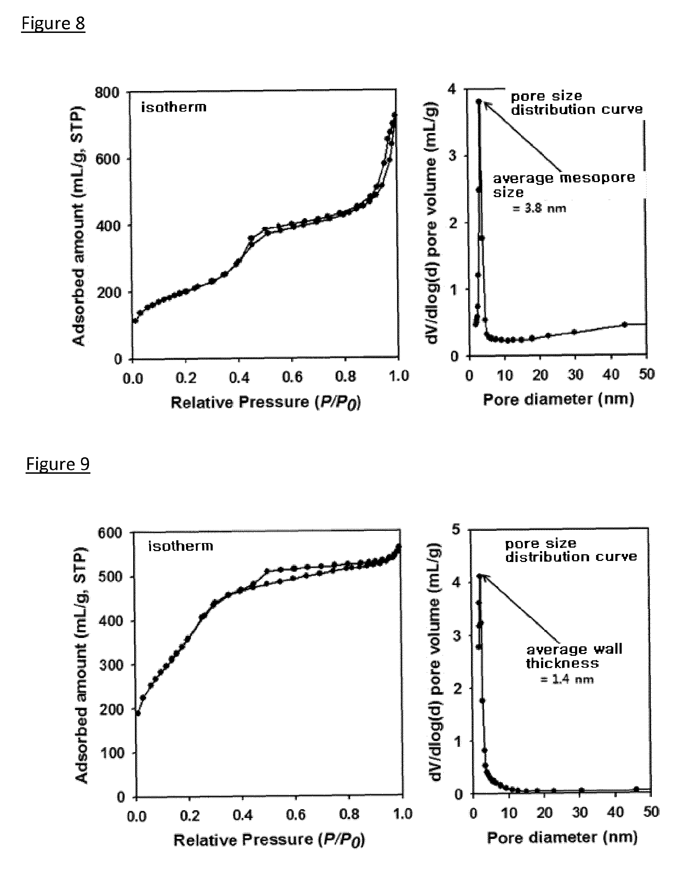

Scanning electron microscope (SEM) images of the material synthesized as described above show that the zeolite is comprised of grown crystals having a thickness of nanometers (5-20 nm) (FIG. 1). FIG. 2 is a set of transmission electron microscope (TEM) images of the cross-section of such zeolite nano-crystals and shows that mesopores having a size of about 3.8 nm were hexagonally arranged and the framework of the structure consisted of a 1-nm-thick zeolite framework including micropores. FIG. 3 shows the low-angle powder X-ray diffraction pattern of the obtained material, and as can be seen therein, typical peaks (100, 110 and 200) corresponding to hexagonal mesoporous structures appeared. This suggests that the obtained material has a hexagonal mesoporous structure. The peak of 100 corresponding to a primary peak appeared at around 1.8°. As can be seen in a high-angle X-ray diffraction pattern (FIG. 4), the synthesized material was comprised of the framework of the crystalline MFI zeolite (hereinafter, this material is referred to as a hexagonally ordered mesoporous MFI aluminosilicate). The ²⁹Si MAS NMR spectrum (FIG. 5) of the zeolite shows a peak corresponding to a chemical shift of about -113 ppm, suggesting that the framework of the material is a crystalline zeolite framework comprised of tetrahedrally coordinated Si (Q⁴). In addition, the peak corresponding to the chemical shift around -103 ppm suggests that Si corresponding to Q³ exists, suggesting that the material includes a significantly large amount of silanol distributed on the surface and has a large outer surface area. The ²⁷Al MAS NMR spectrum (FIG. 6) of the material shows a peak corresponding to a chemical shift of 57-65 ppm, which coincides with the chemical shift of tetrahedrally coordinated Al which is shown in crystalline zeolite structures. NMR peaks of 0-10 ppm corresponding to Al (octahedrally coordinated) present outside the zeolite framework were not observed. The results of argon adsorption analysis (FIG. 7) show that the material was comprised of a zeolite framework including micropores having a size of 0.55 nm, which is consistent with the micropore size of microporous MFI zeolite. In addition, a pore size distribution curve (FIG. 8) obtained by nitrogen adsorption shows that mesopores having a size of 3.8 nm were very uniformly arranged. It was shown that the zeolite material had a BET specific surface area of 800 m²/g and a total pore volume of 1.0 cc/g. FIG. 9 shows the nitrogen adsorption isotherm and pore size distribution curve obtained by filling carbon into the mesopores of the zeolite and then removing the zeolite backbone from a hydrofluoric acid (HF) solution, followed by analysis. The pore size distribution curve of a carbon material which is a replica of the zeolite framework shows that the carbon material had a very uniform size of 1.4 nm, suggesting that the zeolite framework has a very uniform thickness of 1.4 nm. The Si/Al ratio of the product was 29 as measured by ICP (inductively coupled plasma) analysis.

### Example 2: Synthesis of hexagonally ordered mesoporous

### MFI aluminosilicate

It was found that, when a 22-3N-18 organic surfactant was used instead of the 18-3N-18 organic surfactant used in Example 1, a hexagonally ordered MFI aluminosilicate having a decreased mesopore size and the same structure obtained in Example 1 could be synthesized. The 18-3N-18 organic surfactant was mixed with TEOS, NaOH, NaAlO₂ and distilled water to prepare a gel mixture having the following molar composition:
0.5 Al₂O₃: 6.67 Na₂O: 30 SiO₂: 1066 H₂O: 1.3 18-3N-18 organic surfactant.

After strongly stirring the gel mixture with a stirrer at 60 °C for 10 hours, the resulting mixture was placed and maintained in an autoclave at 140 □ for 3 days. After cooling the autoclave to room temperature, the product was filtered and washed several times with distilled water. The resulting mixture was dried at 110 □ and then calcined at 550 °C for 4 hours to remove the organic surfactant.

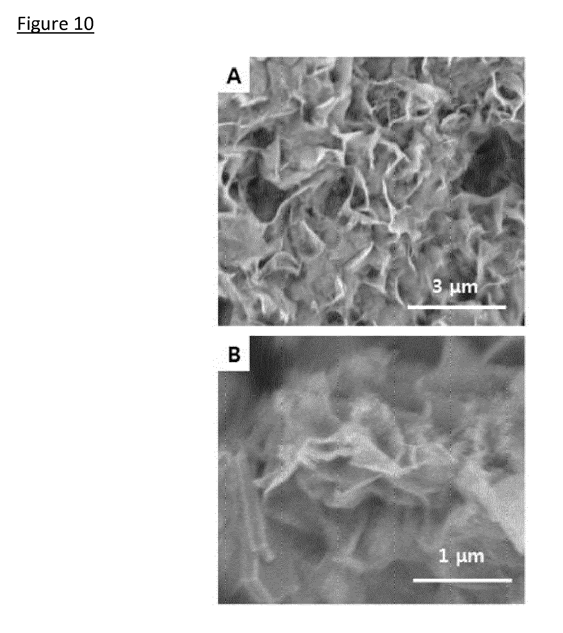

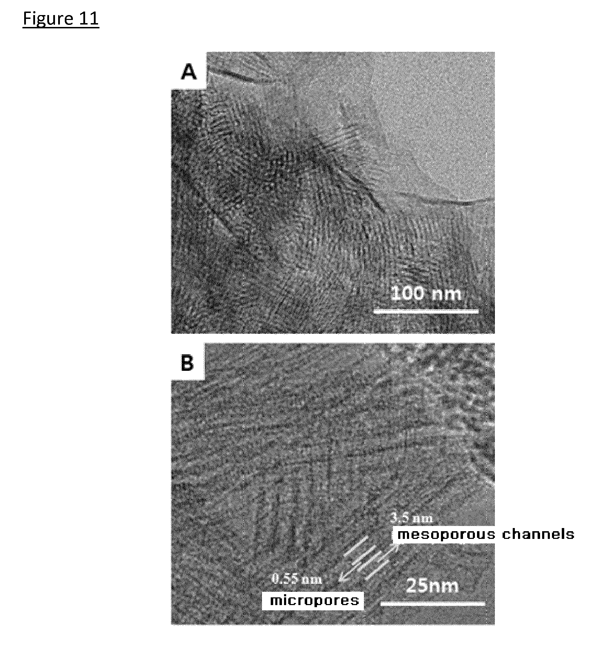

The scanning electron microscope (SEM) images of the synthesized zeolite show that the zeolite was comprised of grown crystals having a thickness of nanometers (5-20 nm) (FIG. 10). FIG. 11 is a set of transmission electron microscope (TEM) images of the cross-section of such zeolite nano-crystals and shows that mesopores having a size of about 3.5 nm were hexagonally ordered and the framework of the structure consisted of a 1 nm thick zeolite framework including micropores.

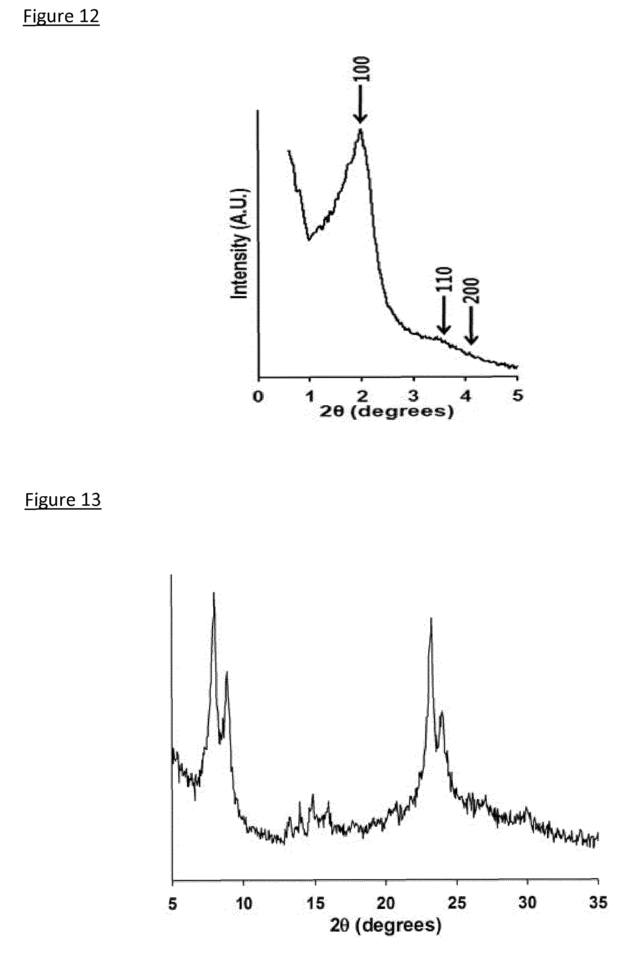

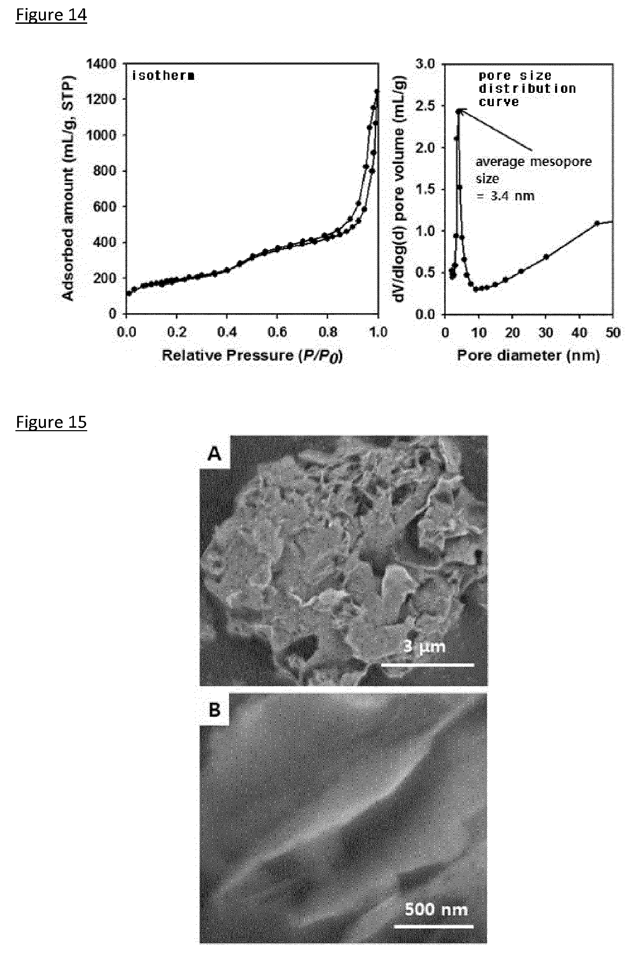

The low-angle X-ray diffraction pattern (FIG. 12) of the material shows peaks indicating a hexagonally ordered structure as shown in Example 1. Unlike the low-angle X-ray diffraction pattern obtained in Example 1, a peak of 100 corresponding to the primary peak appeared at around 1.9°. This suggests that the mesopore size of the material is smaller than that of the material obtained in Example 1. As can be seen in a high-angle X-ray diffraction pattern (FIG. 13), the material was comprised of a crystalline zeolite framework having the MFI structure, like the material obtained in Example 1. The results of nitrogen adsorption analysis (FIG. 14) indicate that mesopores having a very uniform size of about 3.4 nm together with micropores were arranged in the material. It was shown that the zeolite material had a BET specific surface area of 750 m²/g and a total pore volume of 0.9 cc/g. The Si/Al ratio of the product was 26 as measured by ICP analysis.

### Example 3: Synthesis of hexagonally ordered mesoporous MFI aluminosilicate

It was found that, even when a 22-3N-18(OH⁻) organic surfactant having OH⁻ as a counteranion was used instead of the 22-3N-18 organic surfactant (having Br⁻ as a counteranion) used in Example 1, a hexagonally ordered mesoporous MFI aluminosilicate as obtained in Example 1 could be synthesized. The 22-3N-18(OH⁻) organic surfactant was mixed with TEOS, aluminum isopropoxide (Al(*i*OPr)₃) and distilled water without NaOH to prepare a gel mixture having the following molar composition:
0.5 Al₂O₃: 30 SiO₂: 800 H₂O: 1.4 22-3N-18 (OH⁻) organic surfactant.

After strongly stirring the gel mixture with a stirrer at room temperature, the resulting mixture was placed and maintained in an autoclave at 140 □ for 3 days. After cooling the autoclave to room temperature, the product was filtered and washed several times with distilled water. The obtained product was dried at 110 □ and then calcined at 550 °C for 4 hours to remove the organic surfactant.

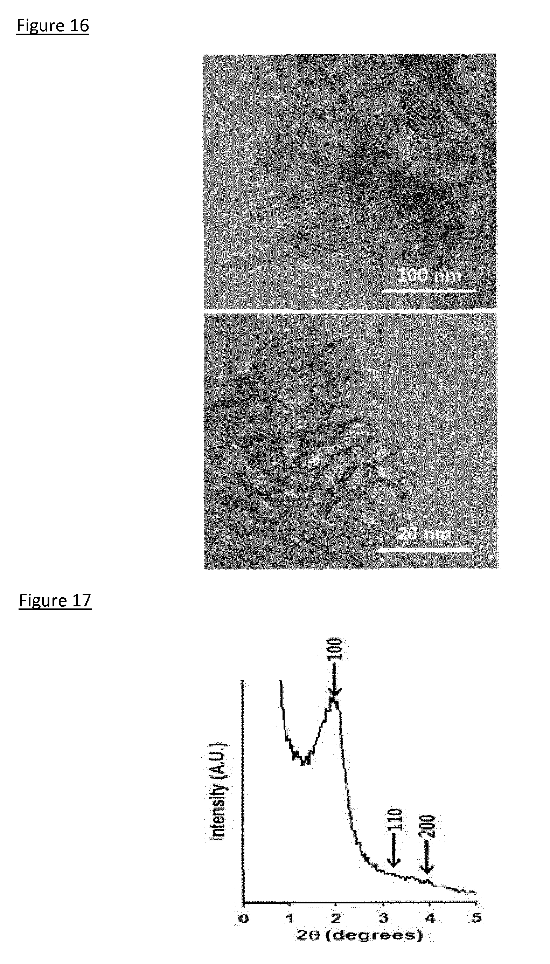

The scanning electron microscope (SEM) images of the synthesized zeolite show that the zeolite was comprised of grown crystals having a thickness of nanometers (5-20 nm) (FIG. 15). FIG. 16 is a set of transmission electron microscope (TEM) images of the cross-section of such zeolite nano-crystals and shows that mesopores having a size of about 3.8 nm were hexagonally ordered and the framework of the structure consisted of a 1 nm thick zeolite framework including micropores.

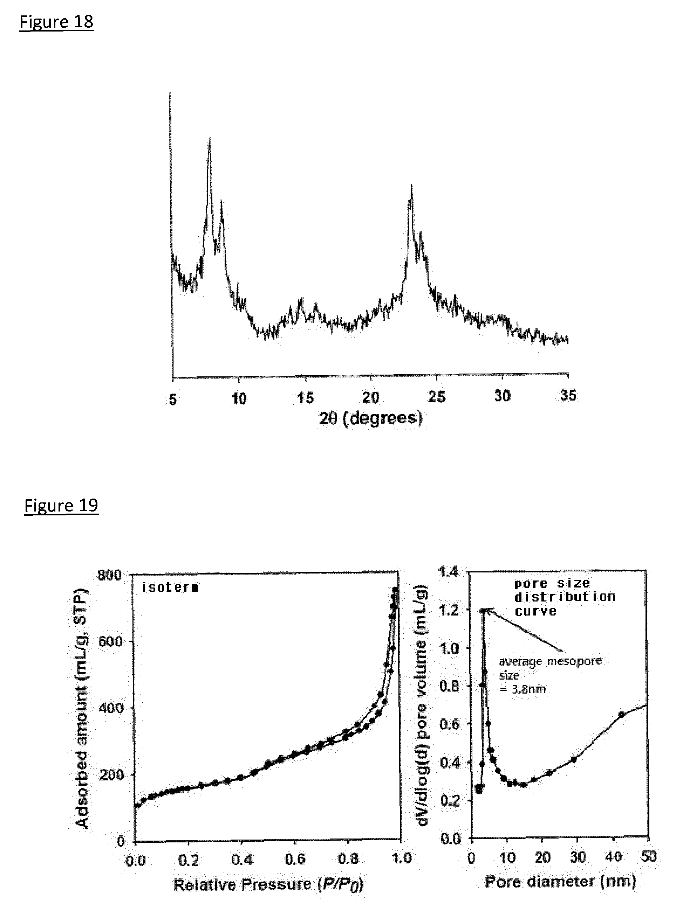

The low-angle X-ray diffraction pattern (FIG. 17) of the material shows peaks indicating a hexagonally ordered structure as shown in Example 1. As can be seen in a high-angle X-ray diffraction pattern (FIG. 18), the synthesized material was comprised of a crystalline zeolite framework having the same structure obtained in Example 1. The results of nitrogen adsorption analysis (FIG. 19) indicate that mesopores having a very uniform size of about 3.8 nm together with micropores are arranged in the synthesized material. It was found that the zeolite material has a BET specific surface area of 720 m²/g and a total pore volume of 1.0 cc/g. The Si/Al ratio of the product was 27 as measured by ICP analysis.

### Example 4: Synthesis of hexagonally ordered mesoporous MFI pure silicate

When aluminum was excluded from a composition for synthesizing the hexagonally ordered mesoporous MFI aluminosilicate using the 22-3N-18 organic surfactant in Example 1, an MFI silicate having the same structure and consisting of silica could be synthesized. The 22-3N-18 organic surfactant was mixed with TEOS, NaOH and distilled water to prepare a gel mixture having the following molar composition:
6.67 Na₂O: 30 SiO₂: 1066 H₂O: 1.3 22-3N-18 organic surfactant.

After strongly stirring the gel mixture with a stirrer at 60 °C for 3 hours, the resulting mixture was placed and maintained in an autoclave at 140 □ for 3 days. After cooling the autoclave to room temperature, the product was filtered and washed several times with distilled water. The obtained product was dried at 110 □ and then calcined at 550 °C for 4 hours to remove the organic surfactant.

The low-angle X-ray diffraction pattern of the silicate material thus synthesized shows peaks corresponding to the same hexagonal structure as the material obtained in Example 1, and the high-angle X-ray diffraction pattern of the material shows that the material coincided with the structure of the highly crystalline microporous MFI molecular sieve obtained in Example 1. It was shown that the zeolite material had a large BET specific surface area of 740 m²/g and was comprised of pure silicate, as determined by ICP analysis.

### Example 5: Synthesis of hexagonally ordered mesoporous MFI titanosilicate

A gel mixture for synthesizing an MFI titanosilicate was prepared by mixing a 22-3N-18(OH-) organic surfactant with TEOS, titanium (IV) butoxide and distilled water. The prepared gel mixture had the following molar composition:
0.3 TiO₂: 30 SiO₂: 800 H₂O: 1.4 22-3N-18 (OH⁻) organic surfactant.

After strongly stirring the gel mixture with a stirrer at room temperature, the resulting mixture was placed and maintained in a stainless autoclave at 140 □ for 5 days. After cooling the autoclave to room temperature, the product was filtered and washed several times with distilled water. The obtained product was dried at 110 □ and then calcined at 550 °C for 4 hours to remove the organic surfactant.

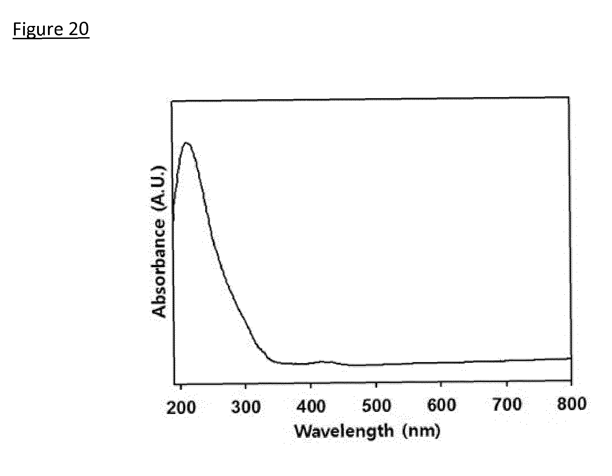

The low-angle X-ray diffraction pattern of the titanosilicate material thus synthesized shows peaks corresponding to the same hexagonal structure as that of the material obtained in Example 1. The high-angle X-ray diffraction pattern of the material shows that the material coincided with the structure of the highly crystalline microporous MFI molecular sieve obtained in Example 1. Also, the results of ultraviolet spectrophotometry of the synthesized material indicate that a high-intensity peak appeared at a wavelength of 220 nm (FIG. 20), suggesting that titanium of the titanosilicate is located at tetrahedrally coordinated silicon. This zeolite material showed a large BET specific surface area of 780 m²/g and had an Si/Ti ratio of 53 as measured by ICP analysis.

### Example 6: Synthesis of hexagonally ordered mesoporous BEA aluminosilicate

When the 22-3N-18 organic surfactant used in Example 1 was used and the composition for synthesis was changed, a hexagonally ordered mesoporous aluminosilicate having a BEA structure, as opposed to the MFI structure, could be synthesized. The 22-3N-18 organic surfactant was mixed with TEOS, NaOH, NaAlO₂ and distilled water to prepare a gel mixture having the following molar composition:
1 Al₂O₃: 6.67 Na₂O: 30 SiO₂: 1066 H₂O: 1.3 22-3N-18 organic surfactant.

After strongly stirring the gel mixture was with a stirrer at 60 °C for 3 hours, the resulting mixture was placed and maintained in a stainless autoclave at 140 □ for 3 days. After cooling the autoclave to room temperature, the product was filtered and washed several times with distilled water. The obtained product was dried at 110 □ and then calcined at 550 °C for 4 hours to remove the organic surfactant.

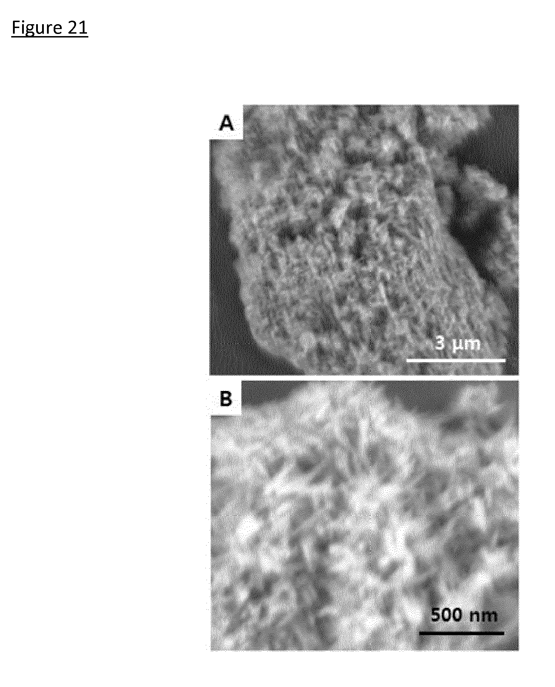

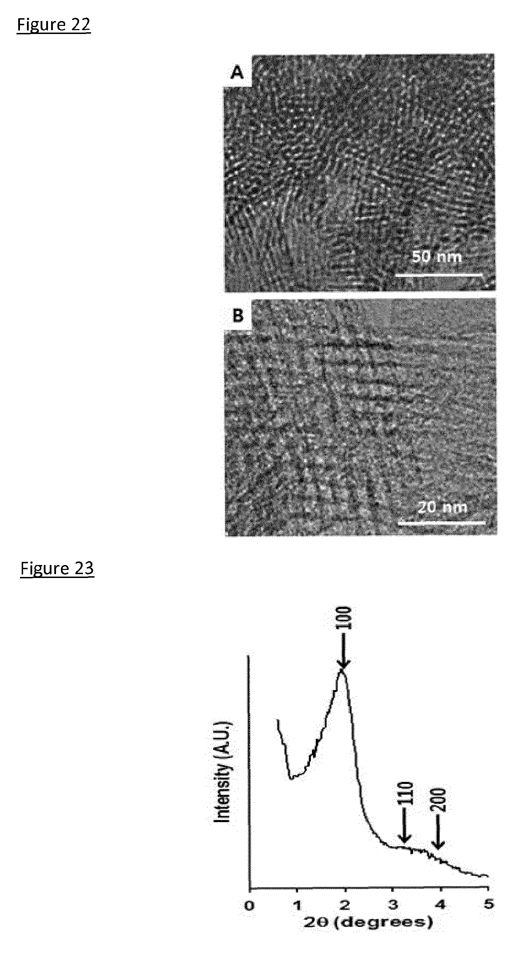

The scanning electron microscope (SEM0 images of the synthesized material show that the material was comprised of grown crystals having a thickness of nanometers (5-20 nm) (FIG. 21). FIG. 22 is a set of transmission electron microscope (TEM) images of such zeolite nano-crystals and shows that mesopores having a size of about 3.7 nm were hexagonally ordered and the framework of the structure consisted of a 1.5 nm thick zeolite framework including micropores.

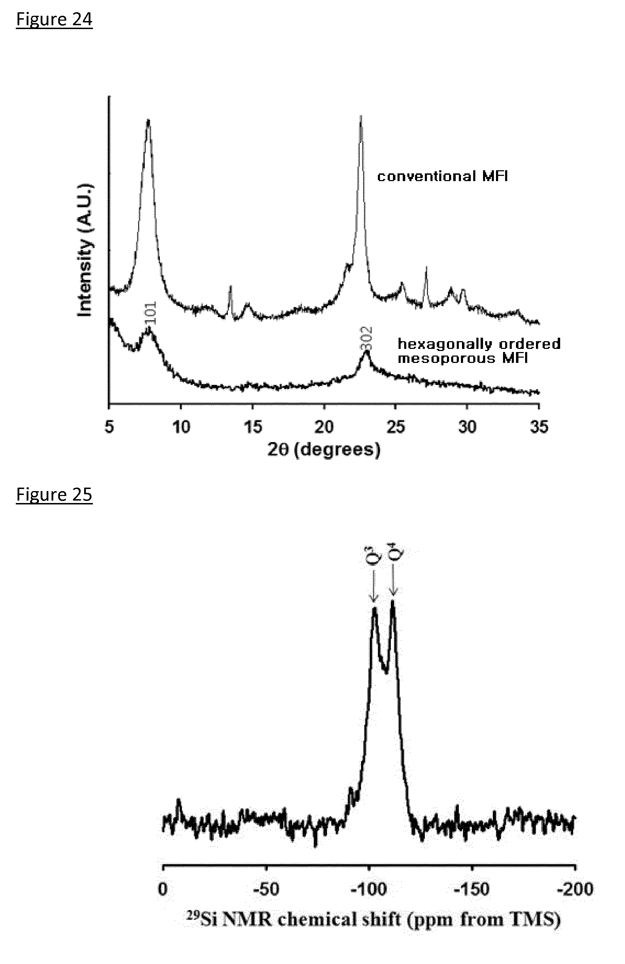

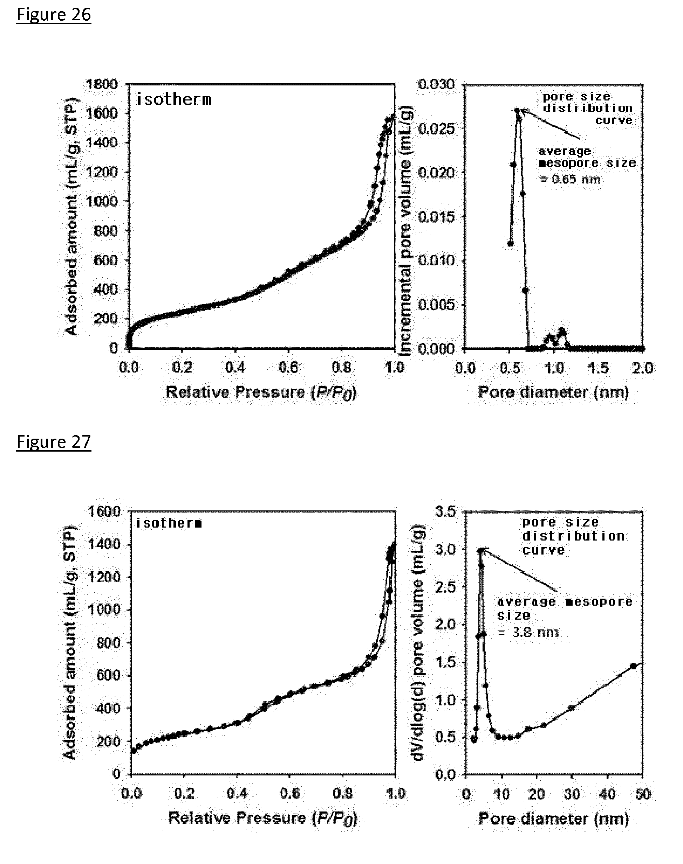

The low-angle X-ray diffraction pattern (FIG. 23) of the material shows the regularity of the mesoporous structure, and as can be seen therein, typical peaks (100, 110, and 200) corresponding to a hexagonal mesoporous structure appeared. This suggests that the material has a hexagonally ordered mesoporous structure. The high-angle X-ray diffraction pattern (FIG. 24) of the material shows that the material was comprised of a crystalline BEA zeolite framework. The ²⁹Si MAS NMR spectrum (FIG. 25) of the zeolite shows a peak corresponding to a chemical shift of about -113 ppm, suggesting that the framework of the material is a crystalline zeolite framework consisting of tetrahedrally coordinated Si (Q⁴). Also, the peak corresponding to the chemical shift of about -103 ppm suggests that Si corresponding to Q³ exists, indicating that the material includes a significantly large amount of silanol distributed on the surface and has a large outer surface area. The ²⁷Al MAS NMR spectrum of the material shows a peak corresponding to a chemical shift of 57-65 ppm, which is consistent with the chemical shift of tetrahedrally coordinated Al which is shown in crystalline zeolite structures. NMR peaks of 0-10 ppm corresponding to Al (octahedrally coordinated) present outside the framework of the zeolite were not observed. The results of argon adsorption analysis (FIG. 26) indicate that the material was comprised of a zeolite framework including micropores having a size of 0.65 nm, which coincided with the micropore size of microporous BEA zeolite. In addition, the pore size distribution curve (FIG. 27) obtained by nitrogen adsorption analysis shows that mesopores having a very uniform size of 3.8 nm were arranged in the material. It was shown that the zeolite material had a BET specific surface area of 840 m²/g and a total pore volume of 1.2 cc/g. Nitrogen adsorption analysis was performed after filling carbon in the mesopores of the zeolite, carbonizing the zeolite and then removing the zeolite framework from a hydrofluoric acid (HF) solution, and the results of the analysis show that the carbon material had a very uniform size of 1.5 nm, suggesting that the zeolite framework has a very uniform thickness of 1.5 nm. The Si/Al ratio of the product was 13 as measured by ICP analysis.

### Example 7: Synthesis of hexagonally ordered mesoporous aluminophosphate zeolite

A gel mixture for synthesizing aluminophosphate was prepared by mixing a 22-3N-18(OH-) organic surfactant with Al(iOPr)₃ and distilled water and adding phosphoric acid thereto. The prepared gel mixture had the following molar composition:
1 Al₂O₃: 1 P₂O₅: 250 H₂O: 0.5 22-3N-18 (OH⁻) organic surfactant.

After strongly stirring the gel mixture with a stirrer at room temperature, the resulting mixture was placed and maintained in a stainless autoclave at 150 □ for 5 days. After cooling the autoclave to room temperature, the product was filtered and washed several times with distilled water. The obtained product was dried at 110 □ and then calcined at 550 °C for 4 hours to remove the organic surfactant.

The low-angle X-ray diffraction pattern of the aluminosilicate thus synthesized shows peaks corresponding to the same hexagonal structure as that of the material obtained in Example 1. The high-angle X-ray diffraction pattern of the material shows that the material was a highly crystalline microporous molecular sieve. It was shown that the zeolite material had a large BET specific surface area of 830 m²/g together with an Al/P ratio of 1 as measured by ICP analysis.

### Example 8: Synthesis of aluminosilicate wherein crystalline microporous BEA zeolite framework is assembled to form mesopores which are irregularly arranged

A 22-6-(*p*-phenylene)-6-22 organic surfactant (chemical formula: CH₃(CH₂)₂₁-N⁺(CH₃)₂-(CH₂)₆-N⁺(CH₃)₂-(CH₂)-(*p*-phenlylene)-(CH₂)-N⁺(CH₃)₂-(CH₂)₆-N⁺(CH₃)₂-(CH₂)₂₁CH₃·2Cl⁻·2Br⁻) was mixed with TEOS, NaOH, NaAlO₂ and distilled water to prepare a gel mixture having the following molar composition:
1 Al₂O₃: 6.67 Na₂O: 30 SiO₂: 1066 H₂O: 1.5 22-6-(*p-*phenylene)-6-22 organic surfactant.

After stirring the gel mixture at room temperature for 3 hours, the resulting mixture was placed and maintained in a stainless autoclave at 140 □ for 2 days. After cooling the autoclave to room temperature, the product was filtered and washed several times with distilled water. The obtained product was dried at 110 □ and then calcined at 550 °C for 4 hours to remove the organic surfactant.

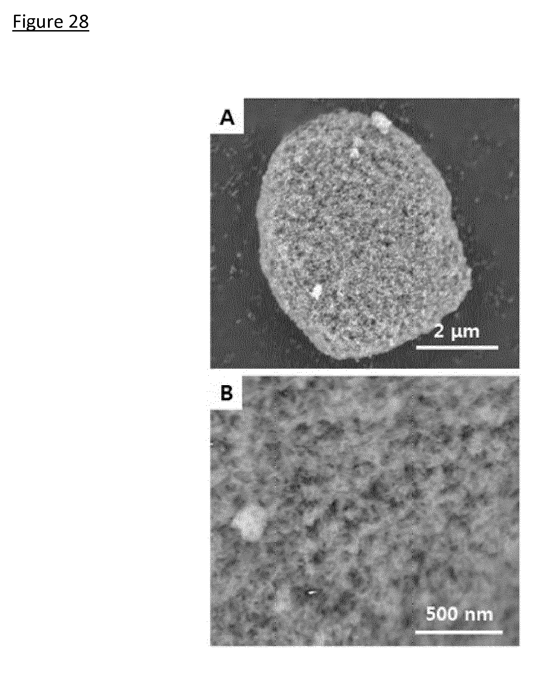

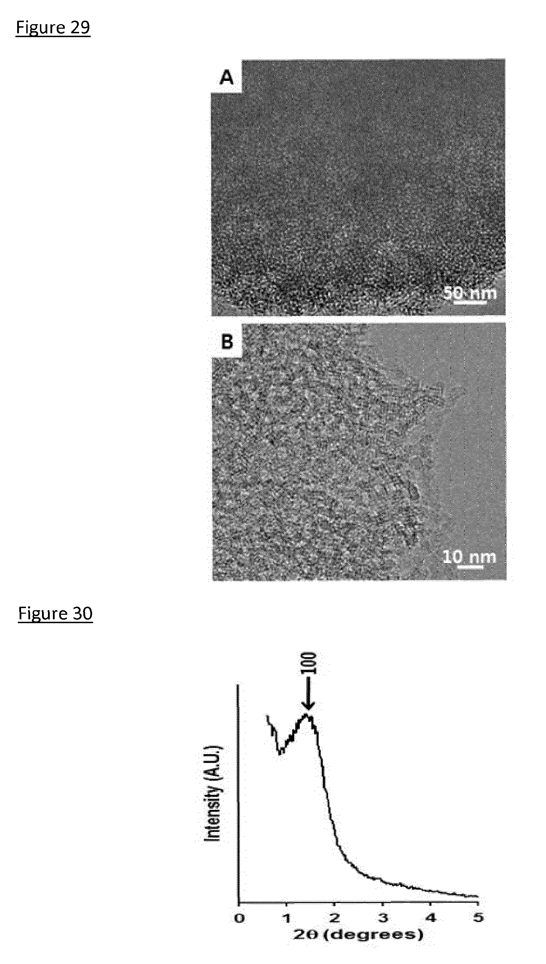

The scanning electron microscope (SEM) images of the zeolite thus synthesized show that the zeolite was comprised of grown crystals having a thickness of nanometers (5-20 nm) (FIG. 28). FIG. 29 is a set of transmission electron microscope (TEM) images of such zeolite nano-crystals and shows that mesopores having a size of about 3.6 nm were irregularly arranged and the framework of the structure consisted of a 2.6 nm thick zeolite framework including micropores.

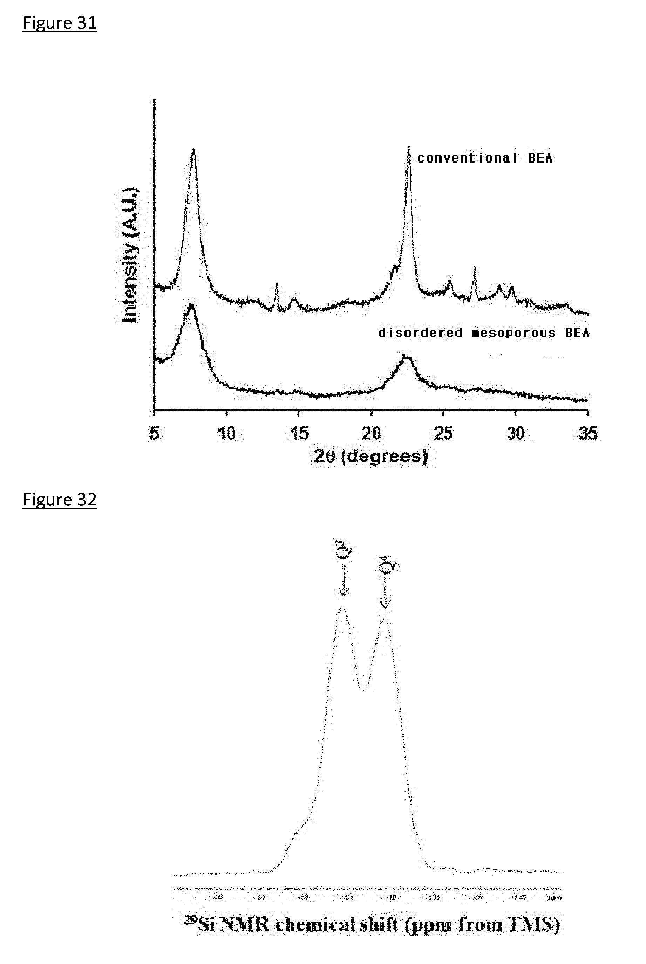

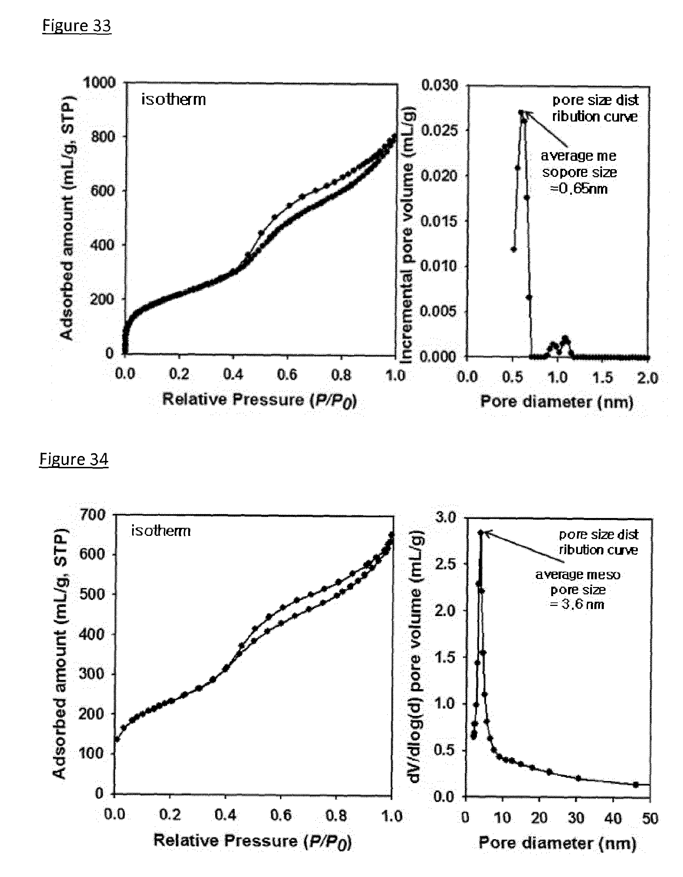

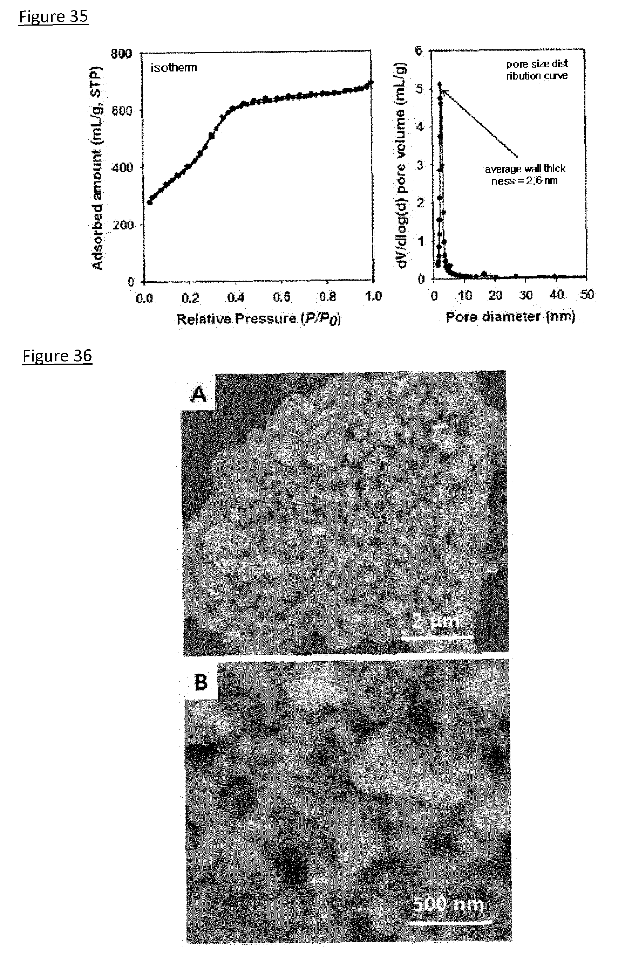

The low-angle X-ray diffraction pattern (FIG. 30) of the material shows the irregularity of the mesoporous structure and showed only a peak corresponding to 100, suggesting that the material has a disordered mesoporous structure. The high-angle X-ray diffraction pattern (FIG. 31) of the material shows that the material consisted of a crystalline BEA zeolite framework (hereinafter, this material is referred to as a disordered BEA aluminosilicate). The ²⁹Si MAS NMR spectrum (FIG. 32) of the zeolite shows a peak corresponding to a chemical shift of about -113 ppm, suggesting that the framework of the material is a crystalline zeolite framework consisting of tetrahedrally coordinated Si (Q⁴). Also, the peak corresponding to a chemical peak of about -103 ppm suggests that Si corresponding to Q³ exists, indicating that the material includes a significantly large amount of silanol distributed on the surface and has a large outer surface area. The ²⁷Al MAS NMR analysis of the material showed a peak corresponding to a chemical shift of 57-65 ppm, which coincides with the chemical shift of tetrahedrally coordinated Al which is shown in crystalline zeolite structures. NMR peaks of 0-10 ppm corresponding to Al (octahedrally coordinated) present outside the zeolite framework were not observed. The results of argon adsorption analysis (FIG. 33) showed that the material was comprised of a zeolite framework including micropores having a size of 0.65 nm, which is consistent with the micropore size of microporous BEA zeolite. Also, the pore size distribution curve (FIG. 34) obtained by nitrogen adsorption analysis shows that mesopores having a very uniform size of 3.6 nm were arranged in the material. It was shown that the zeolite material had a BET specific surface area of 870 m²/g and a total pore volume of 1.3 cc/g. FIG. 35 shows the nitrogen adsorption isotherm and pore size distribution curve obtained by filling carbon filled in the mesopores of the zeolite, carbonizing the zeolite and then removing the zeolite framework from a hydrofluoric acid (HF) solution, followed by analysis. As can be seen in the pore size distribution curve of the carbon material which is a replica of the zeolite framework, the carbon material had a very uniform size of 2.6 nm, indicating that the zeolite framework has a very uniform size of 2.6 nm. The Si/Al ratio of the product was 15 as measured by ICP analysis.

### Example 9: Synthesis of disordered mesoporous BEA aluminosilicate

It was found that, even when a 22-6-(*p*-diphenylene)-6-22 organic surfactant comprising two phenyl groups was used instead of the 22-6-(*p*-phenylene)-6-22 organic surfactant used in Example 8, a disordered mesoporous BEA aluminosilicate as obtained in Example 8 could be synthesized. The 22-6-(*p-*diphenylene)-6-22 organic surfactant was mixed with TEOS, NaOH, NaAlO₂ and distilled water to prepare a gel mixture having the following molar composition:
1 Al₂O₃: 6.67 Na₂O: 30 SiO₂: 1066 H₂O: 1.5 22-6-(*p-*diphenylene)-6-22 organic surfactant.

After stirring the gel mixture at room temperature for 3 hours, the resulting mixture was placed and maintained in a stainless autoclave at 140 □ for 2 days. After cooling the autoclave to room temperature, the product was filtered and washed several times with distilled water. The obtained product was dried at 110 □ and then calcined at 550 °C for 4 hours to remove the organic surfactant.

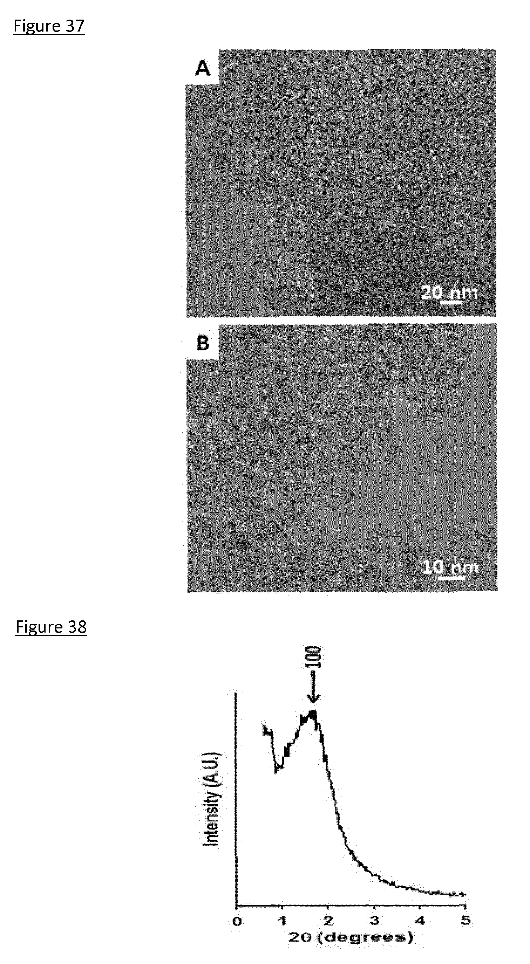

The scanning electron microscope (SEM) images of the zeolite thus synthesized show that the zeolite was comprised of grown crystals having a thickness of nanometers (5-20 nm) (FIG. 36). FIG. 37 is a set of transmission electron microscope (TEM) images of the cross-section of such zeolite nano-crystals and shows that mesopores having a size of about 3.9 nm were irregularly arranged and the framework of the structure consisted of a 3.2 nm thick zeolite framework including micropores.

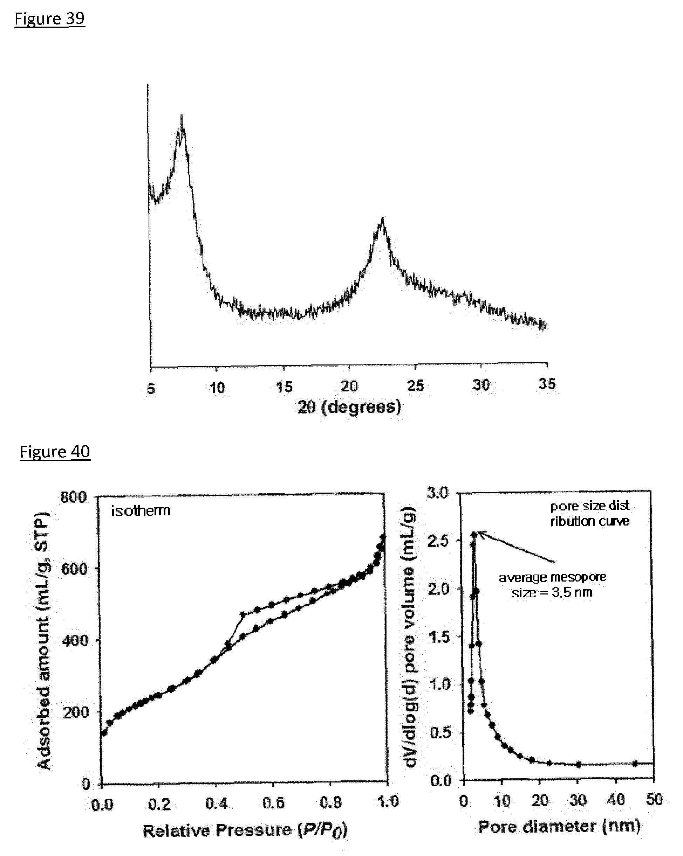

The low-angle X-ray diffraction pattern (FIG. 38) of the material shows peaks indicating a disordered mesoporous structure as shown in Example 8. As can be seen in a high-angle X-ray diffraction pattern (FIG. 39), this material consisted of a crystalline zeolite framework having the same BEA structure as that of the material obtained in Example 8. The results of nitrogen adsorption analysis (FIG. 40) indicated that mesopores having a very uniform size of about 3.5 nm together with micropores were arranged in the material. It was shown that the zeolite material had a BET specific surface area of 880 m²/g and a total pore volume of 1.3 cc/g. The Si/Al ratio of the product was 14 as measured by ICP analysis.

### Example 10: Synthesis of disordered mesoporous BEA aluminosilicate

It was found that, when a 22-6-(*p*-phenylene)-6-22(OH⁻) organic surfactant having OH⁻ as a counteranion was used instead of the 22-6-(*p*-phenylene)-6-22 organic surfactant (having Br⁻ as a counteranion) used in Example 7, a disordered mesoporous BEA aluminosilicate as obtained in Example 8 could be synthesized. The 22-6-(*p*-phenylene)-6-22(OH⁻) organic surfactant was mixed with TEOS, Al(*i*OPr)₃ and distilled water to prepare a gel mixture having the following molar composition:
1 Al₂O₃: 30 SiO₂: 800 H₂O: 1.4 22-6-(*p*-phenylene)-6-22(OH⁻) organic surfactant.

After strongly stirring the gel mixture with a stirrer at room temperature, the resulting product was placed and maintained in a stainless autoclave at 140 □ for 3 days. After cooling the autoclave to room temperature, the product was filtered and washed several times with distilled water. The obtained product was dried at 110 □ and then calcined at 550 °C for 4 hours to remove the organic surfactant.

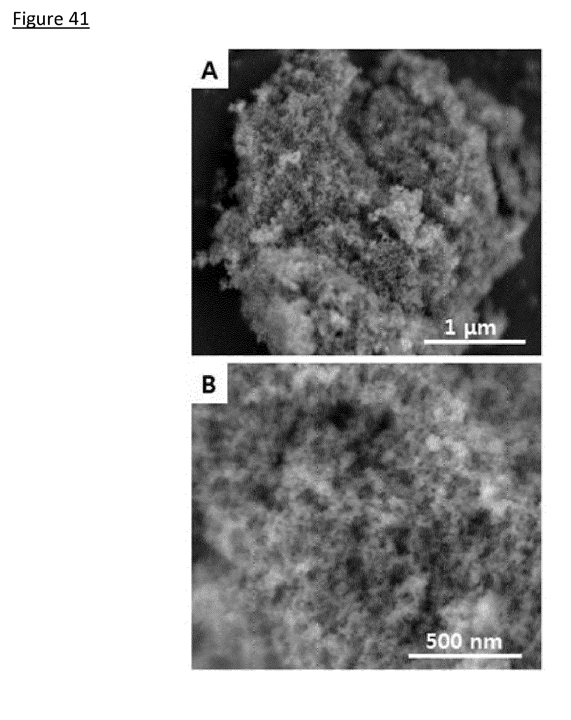

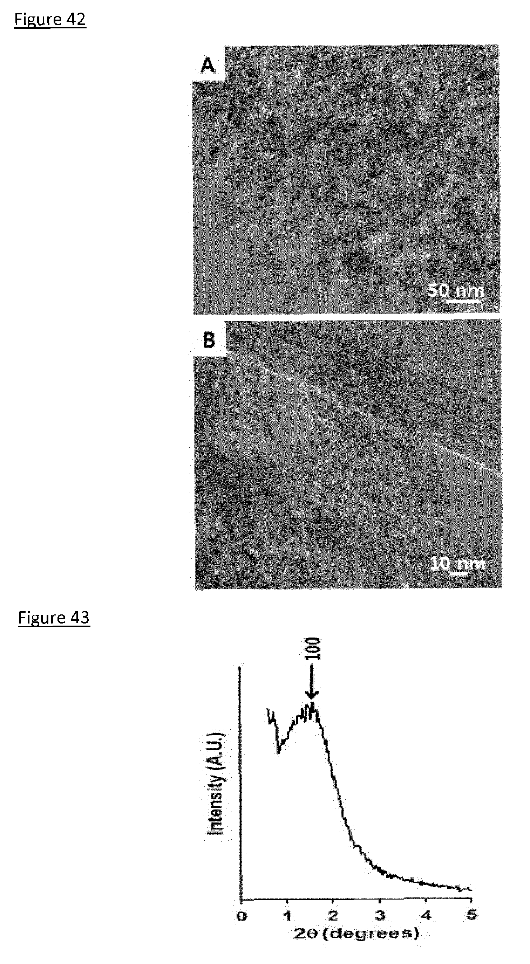

The scanning electron microscope (SEM) images of the zeolite thus synthesized show that the zeolite was comprised of grown crystals having a thickness of nanometers (5-20 nm) (FIG. 41). FIG. 42 is a set of transmission electron microscope (SEM) images of such zeolite nano-crystals and shows that mesopores having a size of about 3.4 nm and micropores having a size of about 48 were irregularly arranged and the framework of the structure consisted of a 3.4 nm thick zeolite framework including micropores.

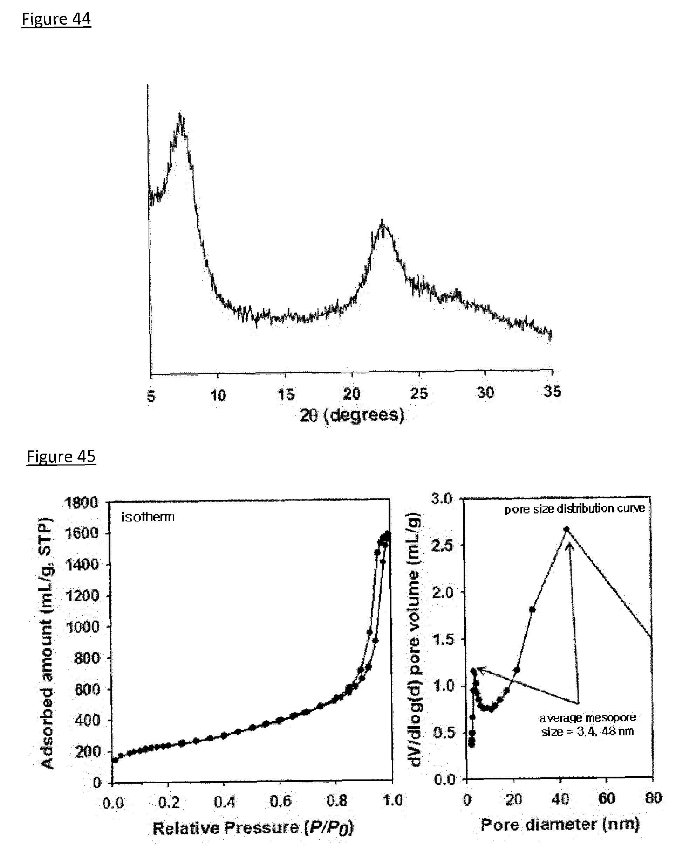

The low-angle X-ray diffraction pattern (FIG. 43) of the zeolite shows peaks indicating a disordered mesoporous structure as shown in Example 8. The high-angle X-ray diffraction pattern (FIG. 44) of the zeolite shows that the material was comprised of a crystalline zeolite framework having a BEA structure, like the material obtained in Example 8. The results of nitrogen adsorption analysis (FIG. 45) indicated that mesopores having different sizes of about 3.4 nm and about 48 nm together with micropores were very uniformly arranged in the material. It was shown that the zeolite material had a BET specific surface area of 850 m²/g and a total pore volume of 2.2 cc/g. Also, the Si/Al ratio of the product was 14 as measured by ICP analysis.

### Example 11: Synthesis of disordered mesoporous BEA silicate

When aluminum was excluded from the composition for synthesizing the BEA aluminosilicate using the 22-6-(*p-*phenylene)-6-22 organic surfactant in Example 8, a BEA silicate consisting only of silica and the same structure could be synthesized. The 22-6-(*p*-phenylene)-6-22 organic surfactant was mixed with TEOS, NaOH and distilled water to prepare a gel mixture having the following molar composition:
13.34 Na₂O: 60 SiO₂: 2132 H₂O: 2.0 22-6-(*p*-phenylene)-6-22 organic surfactant.

After strongly stirring the gel mixture with a stirrer at 60 °C for 3 hours, the resulting mixture was placed and maintained in a stainless autoclave at 140 □ for 3 days. After cooling the autoclave to room temperature, the product was filtered and washed several times with distilled water. The obtained product was dried at 110 □ and then calcined at 550 °C for 4 hours to remove the organic surfactant.

The low-angle X-ray diffraction pattern of the silicate material thus synthesized shows peaks corresponding to the same disordered mesoporous structure as that of the material obtained in Example 8. The high-angle X-ray diffraction pattern of the material indicated that the material coincided with the structure of the highly crystalline microporous BEA molecular sieve obtained in Example 8. It was shown that the zeolite material had a large BET specific surface area of 830 m²/g and consisted only of pure silicate, as measured by ICP analysis.

### Example 12: Synthesis of disordered mesoporous BEA titanosilicate

A gel mixture for synthesizing a BEA titanosilicate was prepared by mixing a 22-6-6-18(OH-) organic surfactant with TEOS, titanium (IV) butoxide and distilled water. The gel mixture had the following molar composition:
0.3 TiO₂: 30 SiO₂: 800 H₂O: 1.4 22-6-(*p*-phenylene)-6-22 (OH⁻) organic surfactant.

After strongly stirring the gel mixture with a stirrer at room temperature, the resulting mixture was placed and maintained in a stainless autoclave at 140 □ for 5 days. After cooling the autoclave to room temperature, the product was filtered and washed several times with distilled water. The obtained product was dried at 110 □ and then calcined at 550 °C for 4 hours to remove the organic surfactant.

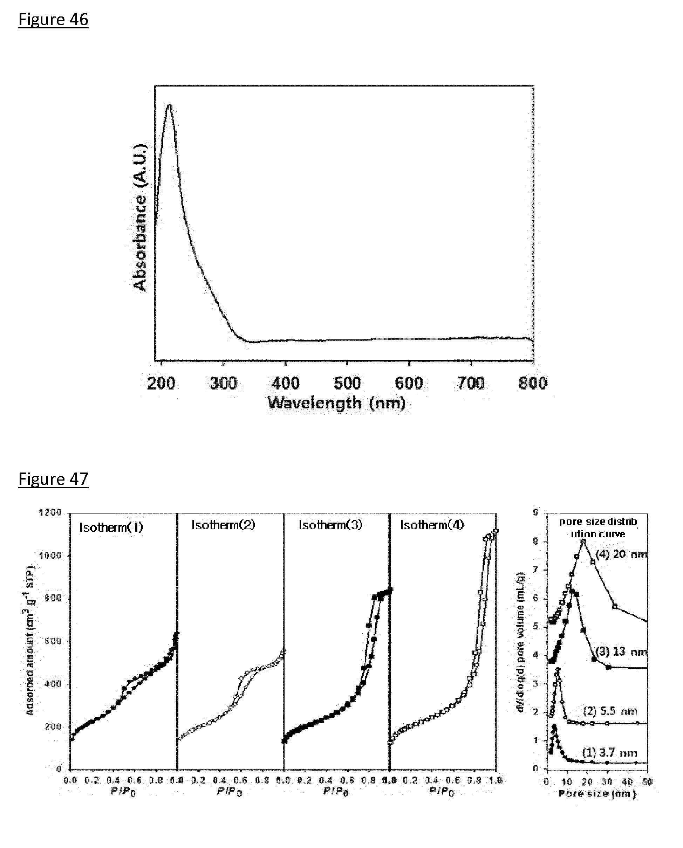

The low-angle X-ray diffraction pattern of the titanosilicate material thus synthesized shows peaks corresponding to the same disordered mesoporous structure as that of the material obtained in Example 8. The high-angle X-ray diffraction pattern of the material shows that the material coincided with the structure of the highly crystalline microporous BEA molecular sieve obtained in Example 8. Also, the results of ultraviolet spectrophotometry of the material indicated that a high-density peak appeared at a wavelength of 220 nm (FIG. 46), suggesting that titanium of the titanosilicate framework is located at tetrahedrally coordinated silicon. The zeolite material showed a large BET specific surface area of 830 m²/g and had an Si/Ti ratio of 59 as measured by ICP analysis.

### Example 13: Synthesis of disordered mesoporous BEA aluminosilicate comprising irregularly arranged mesopores having various sizes by addition of organic solvent

It was found that, when an organic acid such as 1,3,5-trimethylbenzene; 1,3,5-TMB) together with the 22-6-(*p-*phenylene)-6-22 organic surfactant used in Example 7 was added to a gel for zeolite synthesis, a disordered mesoporous BEA aluminosilicate having a significantly increased mesopore size of up to 20 nm could be synthesized. The 22-6-(*p*-diphenylene)-6-22 organic surfactant was mixed with TEOS, NaOH, NaAlO₂, 1,3,5-TMB and distilled water to prepare a gel mixture having the following molar composition:
1 Al₂O₃: 6.67 Na₂O: 30 SiO₂: 1066 H₂O: x 1,3,5-TMB: 1.5 22-6-(*p*-diphenylene)-6-22 organic surfactant (wherein x is 15, 30 or 45).

After stirring the gel mixture at room temperature for 3 hours, the resulting mixture was placed and maintained in a stainless autoclave at 140 □ for 5 days. After cooling the autoclave to room temperature, the product was filtered and washed several times with distilled water. The obtained product was dried at 110 □ and then calcined at 550 °C for 4 hours to remove the organic surfactant.

The zeolite material thus synthesized was calcined at 550 °C for 4 hours to remove the organic surfactant and 1,3,5-TMB and analyzed. The low-angle X-ray diffraction pattern of the zeolite material shows peaks indicating a disordered mesoporous structure as shown in Example 8. As can be seen in a high-angle X-ray diffraction pattern, the material was comprised of a crystalline zeolite framework having the same BEA structure as that of the material obtained in Example 8. The results of nitrogen adsorption analysis (FIG. 47) indicated that micropores together with mesopores were very uniformly arranged in the material. Also, it could be seen that, as the amount of 1,3,5-TMB added increased, the size of mesopores increased gradually from 3.7 nm to 20 nm. In addition, it was shown that the zeolite material had a BET specific surface area of 800-950 m²/g and a total pore volume of 1.0-1.5 cc/g. Further, the Si/Al ratio of the product was 13-15 as measured by ICP analysis.

### Example 14: Synthesis of aluminosilicate wherein crystalline microporous MTW zeolite framework is assembled to form mesopores which are irregularly arranged

The use of the 22-6-(*p*-phenylene)-6-22 organic surfactant used in Example 8 makes it possible to synthesize not only a BEA zeolite structure, but also an aluminosilicate wherein a microporous MTW zeolite framework is assembled to form mesopores which are irregularly arranged. The 22-6-(*p-*phenylene)-6-22 organic surfactant was mixed with TEOS, NaAlO₂,NaOH and distilled water to prepare a gel mixture having the following molar composition:
6.67 Na₂O: 30 SiO₂: 0.3 Al₂O₃: 1066 H₂O: 1.3 22-6-(*p-*phenylene)-6-22 organic surfactant.

After strongly stirring the gel mixture at 60 °C for 3 hours, the resulting mixture was placed and maintained in a stainless autoclave at 140 □ for 3 days. After cooling the autoclave to room temperature, the product was filtered and washed several times with distilled water. The obtained product was dried at 110 □ and then calcined 550 °C for 4 hours to remove the organic surfactant.

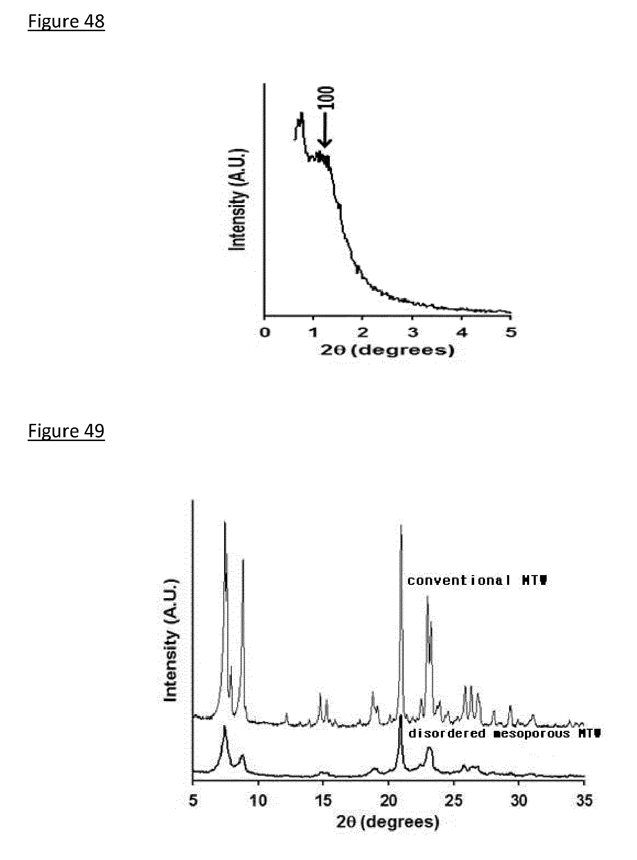

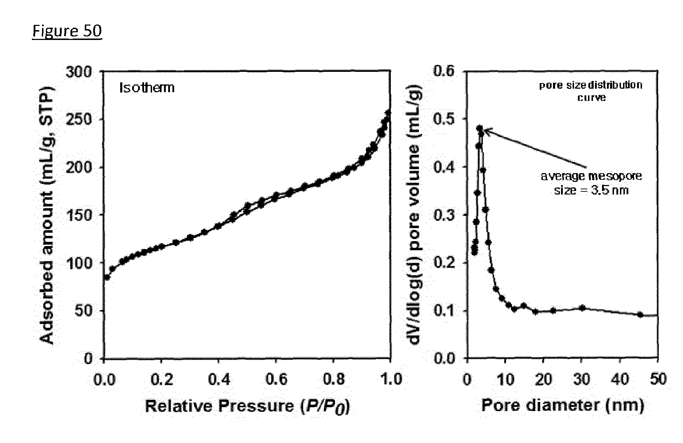

The low-angle X-ray diffraction pattern (FIG. 48) of the silicate material thus synthesized shows peaks corresponding to the same disordered mesoporous structure as that of the material obtained in Example 8. The high-angle X-ray diffraction pattern (FIG. 49) of the material indicates that the material was consistent with the structure of a highly crystalline MTW molecular sieve comprised of a framework having an MTW structure. The results of nitrogen adsorption analysis (FIG. 50) indicated that mesopores having a size of about 3.5 nm together with micropores were very uniformly arranged. It was found that the zeolite material showed a large BET specific surface area of 530 m²/g and had an Si/Al ratio of 47 as measured by ICP analysis.

### Example 15: Dealumination of hexagonally ordered mesoporous MFI and BEA aluminosilicates and disordered mesoporous BEA and MTW aluminosilicates

To 1 g of each of the MFI and BEA aluminosilicates prepared in Examples 1 to 3, 6, 8 to 10, 13 and 14, 40 mL of 2 M oxalic acid was added and stirred under reflux at 65 □ for 1 hour. After completion of the reaction, each of the zeolites was filtered, washed with distilled water, dried at 110 □, and then calcined at 550 □. Table 1 below shows the changes in Si/Al ratio of the dealuminated products, measured by ICP analysis. Meanwhile, the high-angle X-ray diffraction patterns of the MFI and BEA structures were still maintained even after the dealumination reaction.

**Table 1**

| | Example 1 | Example 2 | Example 3 | Example 6 | Example 8 | Example 9 | Example 10 | Example 13 | Example 14 |
|---|---|---|---|---|---|---|---|---|---|
| Before dealumination | 29 | 26 | 27 | 13 | 15 | 14 | 14 | 13-15 | 47 |
| After dealumination | 54 | 49 | 53 | 40 | 42 | 42 | 39 | 38-43 | 82 |

### Example 16: Alkaline treatment of hexagonally ordered mesoporous MFI and BEA zeolites and disordered mesoporous BEA and MTW zeolites

1 g of each of the MFI and BEA zeolites prepared in Examples 1 to 4, 6, 8 to 11, 13 and 14 was added to 100 mL of 0.1 M NaOH solution, and each of the dispersions was stirred for 6 hours, after which the zeolites were filtered, washed with distilled water, and dried at 110 □. The diameters of the mesopores of the MFI and BEA zeolites treated with the alkaline solution increased by about 2-3 nm.

### Example 17: Cation exchange of hexagonally ordered mesoporous MFI and BEA aluminosilicates and disordered mesoporous BEA and MTW aluminosilicates using ammonium nitrate solution

1 g of each of the MFI and BEA aluminosilicates prepared in Examples 1 to 3, 6, 8 to 10, 13 and 14 was added to 40 mL of 1 M ammonium nitrate aqueous solution and stirred under reflux at 50 □ for 5 hours. After completion of the reaction, the zeolites were filtered, washed with distilled water, dried at 110 □, and then calcined at 550 □. The results of ICP analysis of the zeolites indicated that substantially all Na⁺ ions in the micropores of the zeolites were ion-exchanged with H⁺ ions by this process.

### Example 18

Five types of catalytic reaction included in the following example were carried out to show that the present invention is not limited to the hexagonally ordered mesoporous MFI and BEA zeolite and disordered mesoporous BEA and MTW zeolite molecular sieve materials and the preparation methods thereof and that these materials can be applied to various catalytic processes.

### A. Application of hexagonally ordered mesoporous MFI aluminosilicate as catalyst for reforming gaseous methanol

The hexagonally ordered MFI aluminosilicate prepared in Example 1 was exchanged with H⁺- as described in Example 17, and the powder was compressed without a binder. The pellets were ground to obtain molecular sieve particles having a size of 14-20 mesh. To compare the catalytic performance of the zeolites, a conventional MFI zeolite (ZSM-5) was prepared. A methanol reforming reaction was carried out using a fluidized stainless steel reactor (inner diameter: 10 mm, outer diameter = 11 mm, and length = 45 cm) constructed by the present inventors, and the reaction product was analyzed by online gas chromatography connected to the stainless steel reactor. The reaction process was performed as follows. In order to promote the release of reaction heat, 100 mg of the catalyst was mixed with 500 mg of 20-mesh sand and placed in a catalytic device (1/2" filter GSKT-5u) in the stainless reactor. The catalyst was activated at 550 °C for 8 hours under a nitrogen atmosphere, and the temperature of the reactor was lowered to 325 □ (reaction temperature), and then immediately, methanol was introduced into the reactor by a syringe pump at a flow rate of 0.02 mL/m. Herein, the flow rate of nitrogen gas was maintained at 20 mL/m, and the product was periodically analyzed by online gas chromatography. The distribution of the products is shown in Table 2 below. As can be seen therein, the hexagonally ordered MFI aluminosilicate zeolite material of the present invention showed a production distribution significantly different from the conventional MFI catalyst.

**Table 2**

| Product distribution | Hexagonally ordered mesoporous MFI aluminosilicate zeolite (%) | Conventional MFI zeolite (%) |
|---|---|---|
| C₂H₄ | 12.5 | 42.5 |
| C₃H₆ | 48.2 | 0 |
| C₄H₈ | 12.3 | 12.6 |
| Other aliphatic compounds | 24 | 13.1 |
| Benzene | 20 | 26 |
| Toluene | 21 | 14 |
| Xylene | 25 | 8.9 |
| Trimethylbenzene | 42 | 9.2 |
| C₁₀₊ | 12.7 | 9 |
| Others | 11 | 0.7 |
| Total | 100 | 100 |
| Selectivity (%) to olefin | 73.0 | 55.1 |
| Selectivity (%) to gasoline | 23.5 | 31.1 |

### B. Liquid-phase condensation of benzaldehyde with 2-hydroxyaceto-henone

Using the same material used in Example 18A, a catalytic reaction was carried out in a Pyrex reactor equipped with a reflux condenser. Specifically, 0.1 g of the catalyst powder was activated at 180 □ for 2 hours and added to the reactor containing 20 mmol of anhydrous 2-hydroxyacetophenone and 20 mmol of benzaldehyde. The reaction was carried out with stirring at 140 □ in a helium atmosphere. The reaction product was periodically analyzed by gas chromatography. The distribution of the products is shown in FIG. 3 below. As can be seen therein, the MFI zeolite material of the present invention significantly increased catalytic activity compared to the conventional MFI catalyst.

**Table 3**

| Catalyst | Reaction time (hr) | Conversion (%) of 2-hydroxyacetophenone | Product distribution (%) | |
|---|---|---|---|---|
| | | | 2-hydroxychalcon | Flavanone |
| Hexagonally ordered mesoporous MFI aluminosilicate zeolite | 5 | 18.7 | 19.6 | 80.4 |
| | 24 | 50.2 | 15.9 | 84.1 |
| Conventional MFI zeolite | 5 | 4.5 | 6.7 | 93.3 |
| | 24 | 35.6 | 14.6 | 85.4 |

### C. Synthesis of hydrocarbons by reforming of waste plastic

The same material used in Example 18A was used as a catalyst. In this Example, solid powder of unstabilized linear low-density polyethylene was used as a standard reactant. Specifically, a mixture of 10 g of polyethylene and 0.1 g of the catalyst was placed in a semi-batch Pyrex reactor and then physically stirred. The temperature of the reactor was increased from room temperature to 340 □ at a rate of 6 □/m and maintained at that temperature for 2 hours. During the reaction, a volatile product was removed from the reactor using a nitrogen stream (flow rate = 35 mL/m), the product was collected in a liquid phase and a gas form through an ice trap and air pocket attached to the side of the reactor. After completion of the reaction, the liquid-phase and gas-phase products were analyzed by gas chromatography. The distribution of the products is shown in Table 4 below. As can be seen therein, the MFI zeolite material of the present invention showed significantly increased catalytic activity compared to the conventional zeolite material.

**Table 4**

| Catalyst | Conversion (%) | Reaction selectivity (mass%) | | |
|---|---|---|---|---|
| | | C₁-C₅ | C₆-C₁₂ | >C₁₃ |
| Hexagonally ordered mesoporous MFI aluminosilicate zeolite | 81.2 | 89 | 11 | 0 |
| Conventional MFI zeolite | 52.1 | 95 | 5 | 0 |

## Claims

1. A zeolite or zeolite-like material comprising:
a crystalline framework which comprises micropores having a size of 2 nm or less and has a thickness to corresponding to up to 10 single unit cells along at least one axis; and
mesopores formed by self-assembly of the crystalline framework and having a size of 2 nm or more.

2. The zeolite or zeolite-like material of claim 1, wherein the mesopores are hexagonally ordered.

3. The zeolite or zeolite-like material of claim 1, wherein the mesopores are cubically ordered.

4. The zeolite or zeolite-like material of claim 1, wherein the mesopores are disordered.

5. The zeolite or zeolite-like material of claim 1, wherein the crystalline framework includes a metal element selected from the group consisting of Be, B, Al, Ti, Fe, Ga, V, Cr, Co, Ni, Cu, Zn, Ge, Zr, Nb, Sb, La, Hf and Bi.

6. The zeolite or zeolite-like material of claim 1, wherein the crystalline framework has a chemical composition of aluminosilicate, pure silicate, titanosilicate or aluminophosphate.

7. The zeolite or zeolite-like material of claim 1, wherein the zeolite or zeolite-like material has a BET specific surface area of 600-1500 m²/g, a micropore volume of 0.01-0.20 mL/g, and a mesopore volume of 0.1-3.0 mL/g.

8. A material formed by activating or modifying the zeolite or zeolite-like material of any one of claims 1 to 7 using a post-treatment process selected from among dealumination, ion exchange, metal incorporation or organic functionalization.

9. A method for preparing a crystalline molecular sieve, comprising the steps of:
A) polymerizing an organic surfactant of the following formula 1 with an inorganic precursor to form an organic-inorganic hybrid gel comprising nanometer-sized inorganic gel domains stabilized by the organic surfactant;
B) converting the nanometer-sized inorganic gel domains to a zeolite or zeolite-like material by a crystallizing process; and
C) selectively removing the organic surfactant from the material obtained in step B): 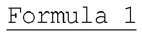 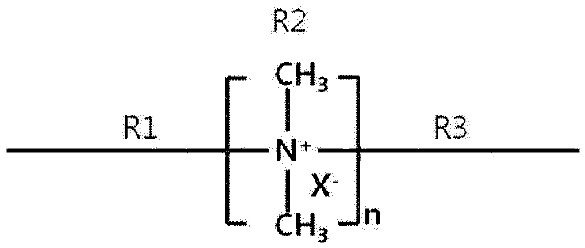 wherein is X⁻ is a halogen anion (Cl⁻, Br⁻, I⁻, etc.) or a hydroxide anion (OH⁻); R1 and R3 are each independently a substituted or unsubstituted alkyl group; R2 is a repeating moiety containing ammonium functional groups; n is the number of ammonium functional groups and is 3 or more; the ammonium functional groups are connected to each other by an alkyl group formed of a hydrocarbon having 3 to 8 carbon atoms; and two methyl (-CH₃) functional groups connected to the ammonium functional group may be substituted with alkyl hydrocarbons having different carbon numbers, such as ethyl (-CH₂CH₃) and propyl (-CH₂CH₂CH₃), or various organic functional groups.

10. The method of claim 9, wherein the inorganic precursor is silica or alumina.

11. The method of claim 9, wherein step A) further comprises adding another surfactant, a polymer, an inorganic salt or an organic additive to control the size of mesopores to in the range of 2-50 nm.

12. The method of claim 9, wherein the crystallizing process is performed using hydrothermal synthesis, microwave heating or dry-gel synthesis.

13. The method of claim 9, wherein the method further comprises a step of activating or modifying the material, obtained in step C), using a post-treatment process selected from dealumination, basic aqueous solution treatment, ion exchange, metal incorporation or organic functionalization.

14. A crystalline molecular sieve prepared by the method of any one of claims 9 to 13.

15. A process comprising catalytically reforming a hydrocarbon or a substituted form thereof using the material of any one of claims 1 to 7 as a catalyst.

16. The process of claim 15, wherein the hydrocarbon is in a gas phase, a liquid phase, a solid phase, or a mixture thereof.

17. A process comprising catalytically reforming a hydrocarbon or a substituted form thereof using the material of claim 8 as a catalyst.

18. The process of claim 17, wherein the hydrocarbon is in a gas phase, a liquid phase, a solid phase, or a mixture thereof.
